# EUROPEAN PATENT APPLICATION

(11) **EP 3 871 675 A1**
(43) Date of publication of application: **01.09.2021**
(21) Application number: 19877003.4
(22) Date of filing: 22.10.2019
(51) Int. Cl.: A61K 31/5377, A61K 31/496, A61P 29/00, A61P 27/04, A61P 37/00

(54) **COMPOSITION FOR PREVENTING OR TREATING SJOGREN'S SYNDROME**

(30) Priority: 23.10.2018 KR 20180126875
(71) Applicant: Samjin Pharmaceutical Co., Ltd., Seoul 04054 (KR)
(72) Inventor: KI, Min-Hyo, Cheonan-si Chungcheongnam-do 31181 (KR); KIM, Hyun-Tae, Suwon-si Gyeonggi-do 16687 (KR); LEE, Jong-Hwan, Seoul 05313 (KR); SON, Yeong-Nam, Seongnam-si Gyeonggi-do 13505 (KR); SHIN, Hee-Jong, Bucheon-si Gyeonggi-do 14574 (KR); LEE, Jung-Rock, Gwangju-si Gyeonggi-do 12767 (KR)
(74) Representative: Maiwald Patent- und Rechtsanwaltsgesellschaft mbH
(86) International application number: PCT/KR2019/013885
(87) International publication number: WO 2020/085760

(57) **Abstract**

The present invention relates to a composition for preventing or treating Sjogren's syndrome. A composition for preventing or treating Sjogren's syndrome according to an embodiment of the present invention includes a compound represented by a specific chemical formula, or a pharmaceutically acceptable salt thereof, and has the effect of preventing or treating Sjogren's syndrome.

## Description

### [Technical Field]

The present invention relates to a composition for preventing or treating Sjogren's syndrome.

### [Background Art]

Sjogren's syndrome is a chronic inflammatory disease of the exocrine glands, and is particularly characterized by a decrease in the production of tears and saliva due to destruction of normal tissues of the salivary glands and lacrimal glands. This disease was first reported by Johann Mikulicz in 1888, and was named after Henrik Sjogren, a Swedish ophthalmologist who described the disease in patients with rheumatoid arthritis in 1933.

Although the main symptom of this disease is decreased eyes and saliva, this disease may also various symptoms by affecting the skin, bronchi, woman's vaginal mucous membrane, lungs and kidneys, in addition to affecting the salivary glands and lacrimal glands.

Sjogren's syndrome that occurs along with other autoimmune diseases, such as Rheumatoid arthritis, systemic lupus erythematosus, scleroderma or dermatomyositis, is referred to as secondary Sjogren's syndrome. Meanwhile, a case in which various symptoms of Sjogren's syndrome appear without a specific rheumatic disease is referred to as primary Sjogren's syndrome. The most common disease with secondary Sjogren's syndrome is rheumatoid arthritis, and about 15% of patients with rheumatoid arthritis have Sjogren's syndrome.

The incidence of Sjogren's syndrome is 9 times higher in women than in men, is particularly higher in middle-aged women, and is estimated to be about 8 per 10,000 women.

Although the cause of this disease has not yet been fully identified, various factors are considered involved in this disease, and genetic factors such as family history, viruses, cytokines, and autoimmune antibodies have been reported as the causes of this disease.

Currently, there is no cure for this disease, and the main purpose of the treatment of this disease is to relieve symptoms and prevent complications. The specific method for treating this disease varies depending on the severity of symptoms such as primary or secondary symptoms.

Korean Patent Application Publication No. 10-2018-0084153 relates to a composition comprising pyrimidine and pyridine compounds having BTK inhibitory activity, and a method for producing these compounds, and discloses that these compounds may be used for the treatment of hyperproliferative diseases, including cancer, lupus, allergic diseases, Sjogren's disease, and rheumatoid arthritis.

Korean Patent Application Publication No. 10-2018-0049144 discloses a nonsteroidal anti-inflammatory agent (NSAIA) prodrug having very high skin and membrane penetration rates and the new pharmaceutical use thereof.
(Patent Document 1) Korean Patent Application Publication No. 10-2018-0084153
(Patent Document 2) Korean Patent Application Publication No. 10-2018-0049144

### [Disclosure]

### [Technical Problem]

An object of the present invention is to provide a composition for preventing or treating Sjogren's syndrome. However, the objects to be solved by the present invention are not limited to the object described above, and other objects that are not described herein will be clearly understood by those skilled in the art from the following description.

### [Technical Solution]

One embodiment of the present invention provides a composition for preventing or treating Sjogren's syndrome, the composition containing a compound represented by the following Formula 1 or a pharmaceutically acceptable salt thereof, 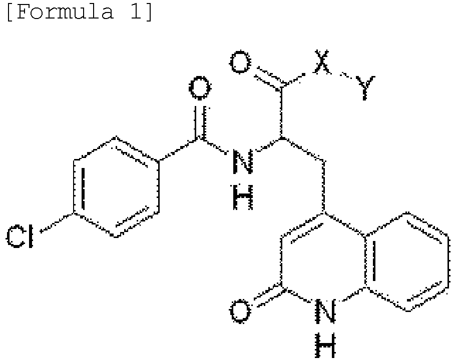 wherein
X is any one of oxygen, nitrogen or sulfur; and
Y is C₁-C₆ alkyl, C₁-C₆ haloalkyl, (C₁-C₃ alkyloxy) C₁-C₆ alkyl, (C₂-C₆ alkenyloxy) C₁-C₆ alkyl, (C₁-C₆ alkylcarbonyloxy) C₁-C₆ alkyl, (C₁-C₆ alkylsulfanyl)C₁-C₆ alkyl, (arylsulfanyl)C₁-C₆ alkyl, (arylsulfonyl) C₁-C₆ alkyl, (C₁-C₆ alkylamino) C₁-C₆ alkyl, [(C₁-C₆ alkyl) (C₁-C₆ alkyl) amino]C₁-C₆ alkyl, [(C₁-C₃ alkyl) (aryl) amino] C₁-C₆ alkyl, [(C₁-C₃ alkyl) (aryl) C₁-C₃ alkyl] amino C₁-C₆ alkyl, [(C₁-C₃ alkyl)(heteroaryl)amino]C₁-C₆ alkyl, (arylcarbonylamino)C₁-C₆ alkyl, C₂-C₆ alkenyl, C₂-C₆ alkynyl, C₂-C₆ oxoalkyl, C₃-C₈ cycloalkyl, (C₃-C₈ cycloalkyl)C₁-C₆ alkyl, (C₃-C₈ cycloalkenyl)C₁-C₆ alkyl, (C₃-C₈ heterocycloalkyl) C₁-C₆ alkyl, [(C₁-C₃ alkyl) C₃-C₈ heterocycloalkyl]C₁-C₆ alkyl, [(aryl)C₁-C₃ alkyl] C₃-C₈ heterocycloalkylC₁-C₆ alkyl, [(C₁-C₆ alkyloxycarbonyl)C₃-C₈ heterocycloalkyl]C₁-C₆ alkyl, [(C₁-C₃ alkyloxycarbonyl)C₃-C₈ heterocycloalkyl] C₁-C₆ alkyl, (C₃-C₈ heterocycloalkyl) C₁-C₆ alkenyl, [(C₁-C₃ alkyl) C₃-C₈ heterocycloalkenyl] C₁-C₆ alkyl, (aryl) C₁-C₆ alkyl, [(C₁-C₃ alkyl) aryl] C₁-C₆ alkyl, [(C₁-C₃ alkyloxy) aryl] C₁-C₆ alkyl, [(aryloxy)aryl]C₁-C₆ alkyl, [(C₁-C₃ alkylsulfanyl)aryl]C₁-C₆ alkyl, [(C₁-C₃ alkyloxycarbonyl)aryl]C₁-C₆ alkyl, [(aryloxycarbonyl) aryl]C₁-C₆ alkyl, (aryl)C₃-C₆ alkenyl, (heteroaryl)C₁-C₆ alkyl, [(alkyloxycarbonyl)heteroaryl]C₁-C₆ alkyl, [(C₁-C₃ alkyl) C₃-C₈ heteroaryl]C₁-C₆ alkyl, [(C₃-C₈ cycloalkyl)heteroaryl]C₁-C₆ alkyl, [(aryl)heteroaryl]C₁-C₆ alkyl, [(C₁-C₃ alkyl) heteroaryl]C₁-C₆ alkyl, [(aryl)C₁-C₃ alkyl]heteroaryl C₁-C₆ alkyl, (C₁-C₆ alkyloxycarbonyl) C₁-C₆ alkyl, [(C₃-C₈ heterocycloalkyl) C₁-C₆ alkyloxycarbonyl]C₁-C₆ alkyl, (C₃-C₈ heterocycloalkylcarbonyl)C₁-C₆ alkyl, [(C₁-C₃ alkyl)C₃-C₈ heterocycloalkylcarbonyl]C₁-C₆ alkyl, [(C₁-C₃ alkyl)C₃-C₈ heterocycloalkylcarbonyl]C₁-C₆ alkyl, [(C₃-C₈ cycloalkyl) oxycarbonyloxy]C₁-C₆ alkyl, [(C₃-C₈ heterocycloalkyl)oxycarbonyloxy]C₁-C₆ alkyl, (ureido)C₁-C₆ alkyl, (arylureido)C₁-C₆ alkyl, [(aryl) (C₁-C₃ alkylureido]C₁-C₆ alkyl, (C₁-C₆ alkylaminocarbonyl) C₁-C₆ alkyl, [(C₃-C₈ heterocycloalkyl)aminocarbonyl]C₁-C₆ alkyl, [(C₁-C₃ alkyl)C₃-C₈ heterocycloalkyl]aminocarbonyl C₁-C₆ alkyl, [(C₁-C₃ alkyl) (C₁-C₃ alkyloxy) aminocarbonyl] C₁-C₆ alkyl, or (oxo C₃-C₈ heterocycloalkyl) C₁-C₆ alkyl,
wherein the C₁-C₆ alkyl, C₂-C₆ alkenyl, C₂-C₆ alkynyl, C₂-C₆ oxoalkyl, C₃-C₈ cycloalkyl, C₃-C₈ cycloalkenyl, C₃-C₈ heterocycloalkenyl, aryl or heteroaryl may be substituted with at least one substituent selected from the group consisting of C₁-C₃ alkyl, fluoro, chloro, bromo, hydroxy, oxo, nitro and cyano groups.

According to one embodiment of the present invention, the salt may be an acid addition salt formed by a pharmaceutically acceptable free acid.

According to one embodiment of the present invention, the free acid may be an organic acid or an inorganic acid.

According to one embodiment of the present invention, the organic may be selected from the group consisting of citric acid, acetic acid, lactic acid, tartaric acid, maleic acid, fumaric acid, formic acid, propionic acid, oxalic acid, dichloroacetic acid, trifluoroacetic acid, adipic acid, ascorbic acid, benzoic acid, 4-acetamidobenzoic acid, gluconic acid, sulfonic acid, methanesulfonic acid, capric acid, capronic acid, caprylic acid, carboxylic acid, cinnamic acid, cyclamic acid, dodecylsulfonic acid, ethane-1,2-disulfonic acid, ethanesulfonic acid, 2-hydroxyethanesulfonic acid, galactaric acid, gentisic acid, glutaric acid, 2-oxoglutaric acid, glycerophosphoric acid, hippuric acid, oleic acid, orotic acid, palmitic acid, pamoic acid, pyroglutamic acid, sebacic acid, stearic acid, thiocyanic acid, undecylenic acid, isobutyric acid, lauric acid, mandelic acid, naphthalene-1,5-disulfonic acid, naphthalene-2-sulfonic acid, naphthoic acid, nicotinic acid, glycolic acid, succinic acid, 4-toluenesulfonic acid, camphorsulfonic acid, glutamic acid, aspartic acid, salicylic acid, 4-aminosalicylic acid, malonic acid, malic acid, and benzosulfonic acid, and the inorganic acid may be selected from the group consisting of hydrochloric acid, bromic acid, sulfuric acid, nitric acid, and phosphoric acid.

According to on embodiment of the present invention, the pharmaceutically acceptable salt of the compound of Formula 1 may be selected from the following:
1) 2-(morpholin-4-yl)ethyl 2-(4-chlorobenzoylamino)-3-(2-oxo-1,2-dihydroquinolin-4-yl)propionate glycolate;
2) 2-(morpholin-4-yl)ethyl 2-(4-chlorobenzoylamino)-3-(2-oxo-1,2-dihydroquinolin-4-yl)propionate lactate;
3) 2-(morpholin-4-yl)ethyl 2-(4-chlorobenzoylamino)-3-(2-oxo-1,2-dihydroquinolin-4-yl)propionate salicylate;
4) 2-(morpholin-4-yl)ethyl 2-(4-chlorobenzoylamino)-3-(2-oxo-1,2-dihydroquinolin-4-yl)propionate oxalate;
5) 2-(morpholin-4-yl)ethyl 2-(4-chlorobenzoylamino)-3-(2-oxo-1,2-dihydroquinolin-4-yl)propionate malonate;
6) 2-(morpholin-4-yl)ethyl 2-(4-chlorobenzoylamino)-3-(2-oxo-1,2-dihydroquinolin-4-yl)propionate maleate;
7) 2-(morpholin-4-yl)ethyl 2-(4-chlorobenzoylamino)-3-(2-oxo-1,2-dihydroquinolin-4-yl)propionate tartrate;
8) 2-(morpholin-4-yl)ethyl 2-(4-chlorobenzoylamino)-3-(2-oxo-1,2-dihydroquinolin-4-yl)propionate maleate;
9) 2-(morpholin-4-yl)ethyl 2-(4-chlorobenzoylamino)-3-(2-oxo-1,2-dihydroquinolin-4-yl)propionate fumarate;
10) 2-(morpholin-4-yl)ethyl 2-(4-chlorobenzoylamino)-3-(2-oxo-1,2-dihydroquinolin-4-yl)propionate citrate;
11) 2-(morpholin-4-yl)ethyl 2-(4-chlorobenzoylamino)-3-(2-oxo-1,2-dihydroquinolin-4-yl)propionate benzenesulfonate;
12) 2-(morpholin-4-yl)ethyl 2-(4-chlorobenzoylamino)-3-(2-oxo-1,2-dihydroquinolin-4-yl)propionate tosylate;
13) 2-(morpholin-4-yl)ethyl 2-(4-chlorobenzoylamino)-3-(2-oxo-1,2-dihydroquinolin-4-yl)propionate hydrochloride;
14) 2-(morpholin-4-yl)ethyl 2-(4-chlorobenzoylamino)-3-(2-oxo-1,2-dihydroquinolin-4-yl)propionate sulfate;
15) 2-(morpholin-4-yl)ethyl 2-(4-chlorobenzoylamino)-3-(2-oxo-1,2-dihydroquinolin-4-yl)propionate phosphrate;
16) 2-morpholin-4-yl-ethyl 2-(4-chlorobenzoylamino)-3-(2-oxo-1,2-dihydroquinolin-4-yl)propionate acetate;
17) 2-morpholin-4-yl-ethyl 2-(4-chlorobenzoylamino)-3-(2-oxo-1,2-dihydroquinolin-4-yl)propionate dichloroacetate;
18) 2-morpholin-4-yl-ethyl 2-(4-chlorobenzoylamino)-3-(2-oxo-1,2-dihydroquinolin-4-yl)propionate adipate;
19) 2-morpholin-4-yl-ethyl 2-(4-chlorobenzoylamino)-3-(2-oxo-1,2-dihydroquinolin-4-yl)propionate ascorbate;
20) 2-morpholin-4-yl-ethyl 2-(4-chlorobenzoylamino)-3-(2-oxo-1,2-dihydroquinolin-4-yl)propionate benzoate;
21) 2-morpholin-4-yl-ethyl 2-(4-chlorobenzoylamino)-3-(2-oxo-1,2-dihydroquinolin-4-yl)propionate 4-acetamidobenzoate;
22) 2-morpholin-4-yl-ethyl 2-(4-chlorobenzoylamino)-3-(2-oxo-1,2-dihydroquinolin-4-yl)propionate caprate;
23) 2-morpholin-4-yl-ethyl 2-(4-chlorobenzoylamino)-3-(2-oxo-1,2-dihydroquinolin-4-yl)propionate capronate;
24) 2-morpholin-4-yl-ethyl 2-(4-chlorobenzoylamino)-3-(2-oxo-1,2-dihydroquinolin-4-yl)propionate caprylate;
25) 2-morpholin-4-yl-ethyl 2-(4-chlorobenzoylamino)-3-(2-oxo-1,2-dihydroquinolin-4-yl)propionate carbonate;
26) 2-morpholin-4-yl-ethyl 2-(4-chlorobenzoylamino)-3-(2-oxo-1,2-dihydroquinolin-4-yl)propionate cinnamate;
27) 2-morpholin-4-yl-ethyl 2-(4-chlorobenzoylamino)-3-(2-oxo-1,2-dihydroquinolin-4-yl)propionate cyclamate;
28) 2-morpholin-4-yl-ethyl 2-(4-chlorobenzoylamino)-3-(2-oxo-1,2-dihydroquinolin-4-yl)propionate dodecylsulfonate;
29) 2-morpholin-4-yl-ethyl 2-(4-chlorobenzoylamino)-3-(2-oxo-1,2-dihydroquinolin-4-yl)propionate ethane-1,2-disulfonate;
30) 2-morpholin-4-yl-ethyl 2-(4-chlorobenzoylamino)-3-(2-oxo-1,2-dihydroquinolin-4-yl)propionate ethanesulfonate;
31) 2-morpholin-4-yl-ethyl 2-(4-chlorobenzoylamino)-3-(2-oxo-1,2-dihydroquinolin-4-yl)propionate 2-hydroxyethanesulfonate;
32) 2-morpholin-4-yl-ethyl 2-(4-chlorobenzoylamino)-3-(2-oxo-1,2-dihydroquinolin-4-yl)propionate formate;
33) 2-morpholin-4-yl-ethyl 2-(4-chlorobenzoylamino)-3-(2-oxo-1,2-dihydroquinolin-4-yl)propionate galactarate;
34) 2-morpholin-4-yl-ethyl 2-(4-chlorobenzoylamino)-3-(2-oxo-1,2-dihydroquinolin-4-yl)propionate gentisate;
35) 2-morpholin-4-yl-ethyl 2-(4-chlorobenzoylamino)-3-(2-oxo-1,2-dihydroquinolin-4-yl)propionate glutamate;
36) 2-morpholin-4-yl-ethyl 2-(4-chlorobenzoylamino)-3-(2-oxo-1,2-dihydroquinolin-4-yl)propionate glutarate;
37) 2-morpholin-4-yl-ethyl 2-(4-chlorobenzoylamino)-3-(2-oxo-1,2-dihydroquinolin-4-yl)propionate 2-oxoglutarate;
38) 2-morpholin-4-yl-ethyl 2-(4-chlorobenzoylamino)-3-(2-oxo-1,2-dihydroquinolin-4-yl)propionate glycerophosphorate ;
39) 2-morpholin-4-yl-ethyl 2-(4-chlorobenzoylamino)-3-(2-oxo-1,2-dihydroquinolin-4-yl)propionate hippurate;
40) 2-morpholin-4-yl-ethyl 2-(4-chlorobenzoylamino)-3-(2-oxo-1,2-dihydroquinolin-4-yl)propionate oleate;
41) 2-morpholin-4-yl-ethyl 2-(4-chlorobenzoylamino)-3-(2-oxo-1,2-dihydroquinolin-4-yl)propionate orotate;
42) 2-morpholin-4-yl-ethyl 2-(4-chlorobenzoylamino)-3-(2-oxo-1,2-dihydroquinolin-4-yl)propionate palmitate;
43) 2-morpholin-4-yl-ethyl 2-(4-chlorobenzoylamino)-3-(2-oxo-1,2-dihydroquinolin-4-yl)propionate pamoate;
44) 2-morpholin-4-yl-ethyl 2-(4-chlorobenzoylamino)-3-(2-oxo-1,2-dihydroquinolin-4-yl)propionate propionate;
45) 2-morpholin-4-yl-ethyl 2-(4-chlorobenzoylamino)-3-(2-oxo-1,2-dihydroquinolin-4-yl)propionate pyroglutamate;
46) 2-morpholin-4-yl-ethyl 2-(4-chlorobenzoylamino)-3-(2-oxo-1,2-dihydroquinolin-4-yl)propionate 4-aminosalicylate;
47) 2-morpholin-4-yl-ethyl 2-(4-chlorobenzoylamino)-3-(2-oxo-1,2-dihydroquinolin-4-yl)propionate sebacate;
48) 2-morpholin-4-yl-ethyl 2-(4-chlorobenzoylamino)-3-(2-oxo-1,2-dihydroquinolin-4-yl)propionate stearate;
49) 2-morpholin-4-yl-ethyl 2-(4-chlorobenzoylamino)-3-(2-oxo-1,2-dihydroquinolin-4-yl)propionate thiocyanate;
50) 2-morpholin-4-yl-ethyl 2-(4-chlorobenzoylamino)-3-(2-oxo-1,2-dihydroquinolin-4-yl)propionate undecylenate;
51) 2-morpholin-4-yl-ethyl 2-(4-chlorobenzoylamino)-3-(2-oxo-1,2-dihydroquinolin-4-yl)propionate hydrobromate;
52) 2-morpholin-4-yl-ethyl 2-(4-chlorobenzoylamino)-3-(2-oxo-1,2-dihydroquinolin-4-yl)propionate isobutyrate;
53) 2-morpholin-4-yl-ethyl 2-(4-chlorobenzoylamino)-3-(2-oxo-1,2-dihydroquinolin-4-yl)propionate laurate;
54) 2-morpholin-4-yl-ethyl 2-(4-chlorobenzoylamino)-3-(2-oxo-1,2-dihydroquinolin-4-yl)propionate DL-mandelate;
55) 2-morpholin-4-yl-ethyl 2-(4-chlorobenzoylamino)-3-(2-oxo-1,2-dihydroquinolin-4-yl)propionate methanesulfonate;
56) 2-morpholin-4-yl-ethyl 2-(4-chlorobenzoylamino)-3-(2-oxo-1,2-dihydroquinolin-4-yl)propionate naphthalene-1,5-disulfonate;
57) 2-morpholin-4-yl-ethyl 2-(4-chlorobenzoylamino)-3-(2-oxo-1,2-dihydroquinolin-4-yl)propionate naphthalene-2-sulfonate;
58) 2-morpholin-4-yl-ethyl 2-(4-chlorobenzoylamino)-3-(2-oxo-1,2-dihydroquinolin-4-yl)propionate 2-naphthoate;
59) 2-morpholin-4-yl-ethyl 2-(4-chlorobenzoylamino)-3-(2-oxo-1,2-dihydroquinolin-4-yl)propionate nicotinate;
60) 2-morpholin-4-yl-ethyl2-(4-chlorobenzoylamino)-3-(2-oxo-1,2-dihydroquinolin-4-yl)propionate nitrate;
61) 2-morpholin-4-yl-ethyl 2-(4-chlorobenzoylamino)-3-(2-oxo-1,2-dihydroquinolin-4-yl)propionate succinate;
62) 2-morpholin-4-yl-ethyl2-(4-chlorobenzoylamino)-3-(2-oxo-1,2-dihydroquinolin-4-yl)propionate sulfonate; and
63) 2-morpholin-4-yl-ethyl 2-(4-chlorobenzoylamino)-3-(2-oxo-1,2-dihydroquinolin-4-yl)propionate L-aspartate.

According to one embodiment of the present invention, the composition may further contain a carrier, an excipient, a diluent, a filler, an anti-aggregating agent, a lubricant, a wetting agent, a flavoring agent, an emulsifying agent or a preservative.

According to one embodiment of the present invention, the composition may be formulated in the form of powder, granule, tablet, emulsion, syrup, aerosol, soft or hard gelatin capsule, sterile injectable solution, or sterile powder.

According to one embodiment of the present invention, the composition may increase tear production and salivary secretion.

According to one embodiment of the present invention, the composition may decrease the mRNA expression levels of immune cell-derived pro-inflammatory cytokines and chemotactic cytokines in lacrimal and salivary gland tissues.

According to one embodiment of the present invention, the composition may activate the survival and growth of cells constituting salivary gland and lacrimal gland tissues.

### [Advantageous Effects]

The composition for preventing or treating Sjogren's syndrome according to an embodiment of the present invention may increase tear production and salivary secretion, and may decrease the mRNA expression levels of immune cell-derived pro-inflammatory cytokines and chemotactic cytokines in lacrimal and salivary gland tissues.

In addition, the composition for preventing or treating Sjogren's syndrome according to one embodiment of the present invention may decrease the expression levels of cytokine proteins in lacrimal and salivary gland tissues, alleviate inflammation, and has the ability to regenerate tissue by increasing the growth of cells. Accordingly, the composition may slow the onset of Sjogren's syndrome, and thus may be used for the purpose of preventing or treating Sjogren's syndrome.

### [Description of Drawings]

FIG. 1 is a graph showing tear production in a disease model of Sjogren's syndrome.
FIG. 2 is a graph showing salivary flow rate in a disease model of Sjogren's syndrome.
FIG. 3 shows the results of measuring the protein expressions of IL-17A and p-AKT and quantifying the same.
FIGS. 4a and 4b show the results of quantifying the mRNAs of pro-inflammatory cytokines and chemotactic cytokines.
FIG. 5 shows the results of measuring the protein expressions of IL-17A and autoantibodies (anti-SSA/Ro, and anti-SSB/La) in plasma.
FIG. 6 depicts photographs showing changes in the inflammatory phenotypes in a disease animal model.
FIG. 7 shows disease scores obtained by scoring phenotypic changes.

### [Best Mode]

Hereinafter, the present invention will be described in detail so that it can be easily carried out by those of ordinary skill in the art to which the present invention pertains.

The present invention may be embodied in a variety of different forms and is not limited to the embodiments and examples described herein.

Throughout the present specification, it is to be understood that when any part is referred to as "including" any component, it does not exclude other components, but may further include other components, unless otherwise specified. As used in the present specification, the term representing the degree such as "about" and "substantially" means that any value is identical or close to a suggested numeral when an inherent fabrication error is proposed, and this is used for preventing any unscrupulous infringer from unfairly using the disclosure containing an exact or absolute numeral, which is mentioned for better understanding of the present disclosure. As used in the entire specification of the present disclosure, the term "step (doing)∼" or a "step of ∼ " does not mean a "step for ∼"

In the present specification, the term "combination thereof" included in Markush-type expressions refers to a mixture or combination of one or more selected from the group consisting of components described by a Markush-type expression, and refers to including one or more selected from the group consisting of the components.

In the present specification, the description of "A and/or B" means "A", "B", or "A and B."

As used in the present invention, the term "heterocycloalkyl" or "heterocycloalkenyl" refers to a cycloalkyl moiety having one or more of nitrogen, oxygen and sulfur as substituent atom(s) at saturated carbon atoms or having the same or different substituent atoms at one, two or three sequential or discontinuous positions. Examples of the heterocycloalkyl or heterocycloalkenyl include, but are not limited to, aziridine, oxirane, azetidine, oxetane, pyrrolidine, pyrroline, pyrazolidine, pyrazoline, imidazolidine, imidazoline, triazolidine, oxazolidine, tetrahydrofuran, tetrahydrothiophene, thiazolidine, dioxolane, dioxole, oxathiolane, morpholine, thiomorpholine, dithiane, piperidine, piperazine, pyran, dioxane, and azepane.

The term "aryl," as used herein, is intended to encompass benzene, naphthalene, anthracene, or phenanthrene, but is not limited thereto. Furthermore, as used herein, the term "heteroaryl" refers to an aryl moiety having one or more of nitrogen, oxygen and sulfur as substituent atom(s) or having the same or different substituent atoms at one, two or three sequential or discontinuous positions. Specific examples of the heteroaryl include, but are not limited to, pyrrole, imidazole, pyrazole, triazole, furan, thiophene, oxazole, isoxazole, thiazole, isothiazole, oxadiazole, thiadiazole, pyridine, pyrimidine, pyrazine, pyridazine, triazine, azepine, indole, benzimidazole, indazole, benzoxazole, benzoisoxazole, benzothiazole, benzotriazole, benzofuran, benzothiophene, quinoline, isoquinoline, quinoxaline, quinazoline, cinnoline, naphthyridine, phthalazine, benzopyran, benzoxazine, benzotriazine, chromane, chromene, benzodioxane, acridine, phenothiazine, phenoxazine, and carbazole.

The present invention relates to a composition for preventing or treating Sjogren's syndrome. The composition for preventing or treating Sjogren syndrome according to the present invention may contain a compound represented by the following Formula 1 or a pharmaceutically acceptable salt thereof.

Sjogren's syndrome is an autoimmune disease in which inflammatory cells infiltrate the lacrimal glands and salivary glands, resulting in chronic inflammation of unknown cause. This disease is known to be caused by loss of control of the immune system, which is the body's defense system. For this reason, inflammatory cells infiltrate the lacrimal glands and salivary glands, which are secretory glands that maintain the humidity of the body, and the normal cells are destroyed, so that the function thereof cannot be maintained anymore. The composition according to one embodiment of the present invention may be administered orally, thereby increasing tear production and salivary secretion, and may decrease the mRNA expression levels of immune cell-derived pro-inflammatory cytokines and chemotactic cytokines in lacrimal and salivary gland tissues. In addition, the composition may decrease the expression levels of cytokine proteins in lacrimal and salivary gland tissues, alleviate inflammation, and has the ability to regenerate tissue by increasing the growth of cells. Accordingly, the composition may slow the onset of Sjogren's syndrome, and thus may be used as a therapeutic agent for Sjogren's syndrome.

Hereinafter, the components and characteristics of the composition for preventing or treating Sjogren's syndrome according to one embodiment of the present invention will be described in more detail.

The composition for preventing or treating Sjogren's syndrome according to one embodiment of the present invention may contain a compound represented by the following Formula 1 or a pharmaceutically acceptable salt thereof: 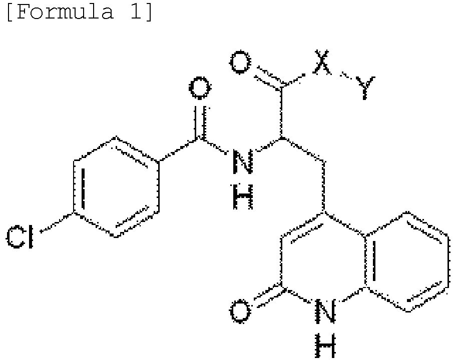 wherein
X is any one of oxygen, nitrogen or sulfur; and
Y is C₁-C₆ alkyl, C₁-C₆ haloalkyl, (C₁-C₃ alkyloxy)C₁-C₆ alkyl, (C₂-C₆ alkenyloxy) C₁-C₆ alkyl, (C₁-C₆ alkylcarbonyloxy) C₁-C₆ alkyl, (C₁-C₆ alkylsulfanyl)C₁-C₆ alkyl, (arylsulfanyl)C₁-C₆ alkyl, (arylsulfonyl) C₁-C₆ alkyl, (C₁-C₆ alkylamino) C₁-C₆ alkyl, [(C₁-C₆ alkyl) (C₁-C₆ alkyl) amino] C₁-C₆ alkyl, [(C₁-C₃ alkyl) (aryl) amino] C₁-C₆ alkyl, [(C₁-C₃ alkyl) (aryl) C₁-C₃ alkyl] amino C₁-C₆ alkyl, [(C₁-C₃ alkyl)(heteroaryl)amino]C₁-C₆ alkyl, (arylcarbonylamino)C₁-C₆ alkyl, C₂-C₆ alkenyl, C₂-C₆ alkynyl, C₂-C₆ oxoalkyl, C₃-C₈ cycloalkyl, (C₃-C₈ cycloalkyl)C₁-C₆ alkyl, (C₃-C₈ cycloalkenyl)C₁-C₆ alkyl, (C₃-C₈ heterocycloalkyl) C₁-C₆ alkyl, [(C₁-C₃ alkyl) C₃-C₈ heterocycloalkyl]C₁-C₆ alkyl, [(aryl)C₁-C₃ alkyl] C₃-C₈ heterocycloalkylC₁-C₆ alkyl, [(C₁-C₆ alkyloxycarbonyl)C₃-C₈ heterocycloalkyl]C₁-C₆ alkyl, [(C₁-C₃ alkyloxycarbonyl)C₃-C₈ heterocycloalkyl] C₁-C₆ alkyl, (C₃-C₈ heterocycloalkyl) C₁-C₆ alkenyl, [(C₁-C₃ alkyl) C₃-C₈ heterocycloalkenyl] C₁-C₆ alkyl, (aryl) C₁-C₆ alkyl, [(C₁-C₃ alkyl) aryl] C₁-C₆ alkyl, [(C₁-C₃ alkyloxy) aryl] C₁-C₆ alkyl, [(aryloxy)aryl]C₁-C₆ alkyl, [(C₁-C₃ alkylsulfanyl)aryl]C₁-C₆ alkyl, [(C₁-C₃ alkyloxycarbonyl)aryl]C₁-C₆ alkyl, [(aryloxycarbonyl) aryl]C₁-C₆ alkyl, (aryl)C₃-C₆ alkenyl, (heteroaryl)C₁-C₆ alkyl, [(alkyloxycarbonyl)heteroaryl]C₁-C₆ alkyl, [(C₁-C₃alkyl) C₃-C₈ heteroaryl]C₁-C₆ alkyl, [(C₃-C₈ cycloalkyl)heteroaryl]C₁-C₆ alkyl, [(aryl)heteroaryl]C₁-C₆ alkyl, [(C₁-C₃ alkyl) heteroaryl]C₁-C₆ alkyl, [(aryl) C₁-C₃ alkyl]heteroaryl C₁-C₆ alkyl, (C₁-C₆ alkyloxycarbonyl) C₁-C₆ alkyl, [(C₃-C₈ heterocycloalkyl) C₁-C₆ alkyloxycarbonyl]C₁-C₆ alkyl, (C₃-C₈ heterocycloalkylcarbonyl)C₁-C₆ alkyl, [(C₁-C₃alkyl)C₃-C₈ heterocycloalkylcarbonyl]C₁-C₆ alkyl, [(C₁-C₃ alkyl)C₃-C₈ heterocycloalkylcarbonyl]C₁-C₆ alkyl, [(C₃-C₈ cycloalkyl) oxycarbonyloxy] C₁-C₆ alkyl, [(C₃-C₈ heterocycloalkyl)oxycarbonyloxy]C₁-C₆ alkyl, (ureido)C₁-C₆ alkyl, (arylureido) C₁-C₆ alkyl, [(aryl) (C₁-C₃ alkylureido]C₁-C₆ alkyl, (C₁-C₆ alkylaminocarbonyl) C₁-C₆ alkyl, [(C₃-C₈ heterocycloalkyl)aminocarbonyl]C₁-C₆ alkyl, [(C₁-C₃ alkyl)C₃-C₈ heterocycloalkyl]aminocarbonyl C₁-C₆ alkyl, [(C₁-C₃ alkyl) (C₁-C₃ alkyloxy) aminocarbonyl] C₁-C₆ alkyl, or (oxo C₃-C₈ heterocycloalkyl) C₁-C₆ alkyl,
wherein the C₁-C₆ alkyl, C₂-C₆ alkenyl, C₂-C₆ alkynyl, C₂-C₆ oxoalkyl, C₃-C₈ cycloalkyl, C₃-C₈ cycloalkenyl, C₃-C₈ heterocycloalkenyl, aryl or heteroaryl may be substituted with at least one substituent selected from the group consisting of C₁-C₃ alkyl, fluoro, chloro, bromo, hydroxy, oxo, nitro and cyano groups.

In the present specification, X-Y represents an amino acid or an amino acid (C₁-C₃ alkyl) ester. The amino acid refers to, but is not limited to, glycine, leucine, methionine, valine, alanine, isoleucine, proline, tryptophan, phenylalanine, serine, threonine, asparagine, glutamic acid, lysine, histidine, or tyrosine.

The composition for preventing or treating Sjogren syndrome according to the present invention was experimentally confirmed to be effective for the prevention or treatment of Sjogren's syndrome by containing the compound represented by Formula 1 or a pharmaceutically acceptable salt thereof.

The compound represented by Formula 1 is a rebamipide precursor, which has an excellent *in vivo* absorption rate compared to rebamipide.

The pharmaceutically acceptable salt of the compound represented by Formula 1 according to one embodiment of the present invention may be an acid addition salt formed by a pharmaceutically acceptable free acid. As the free acid, an organic acid or an inorganic acid may be used. Examples of the organic acid include, but are not limited to, citric acid, acetic acid, lactic acid, tartaric acid, maleic acid, fumaric acid, formic acid, propionic acid, oxalic acid, dichloroacetic acid, trifluoroacetic acid, adipic acid, ascorbic acid, benzoic acid, 4-acetamidobenzoic acid, gluconic acid, sulfonic acid, methanesulfonic acid, capric acid, capronic acid, caprylic acid, carboxylic acid, cinnamic acid, cyclamic acid, dodecylsulfonic acid, ethane-1,2-disulfonic acid, ethanesulfonic acid, 2-hydroxyethanesulfonic acid, galactaric acid, gentisic acid, glutaric acid, 2-oxoglutaric acid, glycerophosphoric acid, hippuric acid, oleic acid, orotic acid, palmitic acid, pamoic acid, pyroglutamic acid, sebacic acid, stearic acid, thiocyanic acid, undecylenic acid, isobutyric acid, lauric acid, mandelic acid, naphthalene-1,5-disulfonic acid, naphthalene-2-sulfonic acid, naphthoic acid, nicotinic acid, glycolic acid, succinic acid, 4-toluenesulfonic acid, camphorsulfonic acid, glutamic acid, aspartic acid, salicylic acid, 4-aminosalicylic acid, malonic acid, malic acid, and benzosulfonic acid. In addition, examples of the inorganic acid include, but are not limited to, hydrochloric acid, bromic acid, sulfuric acid, nitric acid, and phosphoric acid.

According to one embodiment of the present invention, the salt compounds of the rebamipide precursor include, but are not limited to, the following:
1) 2-(morpholin-4-yl)ethyl 2-(4-chlorobenzoylamino)-3-(2-oxo-1,2-dihydroquinolin-4-yl)propionate glycolate;
2) 2-(morpholin-4-yl)ethyl 2-(4-chlorobenzoylamino)-3-(2-oxo-1,2-dihydroquinolin-4-yl)propionate lactate;
3) 2-(morpholin-4-yl)ethyl 2-(4-chlorobenzoylamino)-3-(2-oxo-1,2-dihydroquinolin-4-yl)propionate salicylate;
4) 2-(morpholin-4-yl)ethyl 2-(4-chlorobenzoylamino)-3-(2-oxo-1,2-dihydroquinolin-4-yl)propionate oxalate;
5) 2-(morpholin-4-yl)ethyl 2-(4-chlorobenzoylamino)-3-(2-oxo-1,2-dihydroquinolin-4-yl)propionate malonate;
6) 2-(morpholin-4-yl)ethyl 2-(4-chlorobenzoylamino)-3-(2-oxo-1,2-dihydroquinolin-4-yl)propionate maleate;
7) 2-(morpholin-4-yl)ethyl 2-(4-chlorobenzoylamino)-3-(2-oxo-1,2-dihydroquinolin-4-yl)propionate tartrate;
8) 2-(morpholin-4-yl)ethyl 2-(4-chlorobenzoylamino)-3-(2-oxo-1,2-dihydroquinolin-4-yl)propionate maleate;
9) 2-(morpholin-4-yl)ethyl 2-(4-chlorobenzoylamino)-3-(2-oxo-1,2-dihydroquinolin-4-yl)propionate fumarate;
10) 2-(morpholin-4-yl)ethyl 2-(4-chlorobenzoylamino)-3-(2-oxo-1,2-dihydroquinolin-4-yl)propionate citrate;
11) 2-(morpholin-4-yl)ethyl 2-(4-chlorobenzoylamino)-3-(2-oxo-1,2-dihydroquinolin-4-yl)propionate benzenesulfonate;
12) 2-(morpholin-4-yl)ethyl 2-(4-chlorobenzoylamino)-3-(2-oxo-1,2-dihydroquinolin-4-yl)propionate tosylate;
13) 2-(morpholin-4-yl)ethyl 2-(4-chlorobenzoylamino)-3-(2-oxo-1,2-dihydroquinolin-4-yl)propionate hydrochloride;
14) 2-(morpholin-4-yl)ethyl 2-(4-chlorobenzoylamino)-3-(2-oxo-1,2-dihydroquinolin-4-yl)propionate sulfate;
15) 2-(morpholin-4-yl)ethyl 2-(4-chlorobenzoylamino)-3-(2-oxo-1,2-dihydroquinolin-4-yl)propionate phosphrate;
16) 2-morpholin-4-yl-ethyl 2-(4-chlorobenzoylamino)-3-(2-oxo-1,2-dihydroquinolin-4-yl)propionate acetate;
17) 2-morpholin-4-yl-ethyl 2-(4-chlorobenzoylamino)-3-(2-oxo-1,2-dihydroquinolin-4-yl)propionate dichloroacetate;
18) 2-morpholin-4-yl-ethyl 2-(4-chlorobenzoylamino)-3-(2-oxo-1,2-dihydroquinolin-4-yl)propionate adipate;
19) 2-morpholin-4-yl-ethyl 2-(4-chlorobenzoylamino)-3-(2-oxo-1,2-dihydroquinolin-4-yl)propionate ascorbate;
20) 2-morpholin-4-yl-ethyl 2-(4-chlorobenzoylamino)-3-(2-oxo-1,2-dihydroquinolin-4-yl)propionate benzoate;
21) 2-morpholin-4-yl-ethyl 2-(4-chlorobenzoylamino)-3-(2-oxo-1,2-dihydroquinolin-4-yl)propionate 4-acetamidobenzoate;
22) 2-morpholin-4-yl-ethyl 2-(4-chlorobenzoylamino)-3-(2-oxo-1,2-dihydroquinolin-4-yl)propionate caprate;
23) 2-morpholin-4-yl-ethyl 2-(4-chlorobenzoylamino)-3-(2-oxo-1,2-dihydroquinolin-4-yl)propionate capronate;
24) 2-morpholin-4-yl-ethyl 2-(4-chlorobenzoylamino)-3-(2-oxo-1,2-dihydroquinolin-4-yl)propionate caprylate;
25) 2-morpholin-4-yl-ethyl 2-(4-chlorobenzoylamino)-3-(2-oxo-1,2-dihydroquinolin-4-yl)propionate carbonate;
26) 2-morpholin-4-yl-ethyl 2-(4-chlorobenzoylamino)-3-(2-oxo-1,2-dihydroquinolin-4-yl)propionate cinnamate;
27) 2-morpholin-4-yl-ethyl 2-(4-chlorobenzoylamino)-3-(2-oxo-1,2-dihydroquinolin-4-yl)propionate cyclamate;
28) 2-morpholin-4-yl-ethyl 2-(4-chlorobenzoylamino)-3-(2-oxo-1,2-dihydroquinolin-4-yl)propionate dodecylsulfonate;
29) 2-morpholin-4-yl-ethyl 2-(4-chlorobenzoylamino)-3-(2-oxo-1,2-dihydroquinolin-4-yl)propionate ethane-1,2-disulfonate;
30) 2-morpholin-4-yl-ethyl 2-(4-chlorobenzoylamino)-3-(2-oxo-1,2-dihydroquinolin-4-yl)propionate ethanesulfonate;
31) 2-morpholin-4-yl-ethyl 2-(4-chlorobenzoylamino)-3-(2-oxo-1,2-dihydroquinolin-4-yl)propionate 2-hydroxyethanesulfonate;
32) 2-morpholin-4-yl-ethyl 2-(4-chlorobenzoylamino)-3-(2-oxo-1,2-dihydroquinolin-4-yl)propionate formate;
33) 2-morpholin-4-yl-ethyl 2-(4-chlorobenzoylamino)-3-(2-oxo-1,2-dihydroquinolin-4-yl)propionate galactarate;
34) 2-morpholin-4-yl-ethyl 2-(4-chlorobenzoylamino)-3-(2-oxo-1,2-dihydroquinolin-4-yl)propionate gentisate;
35) 2-morpholin-4-yl-ethyl 2-(4-chlorobenzoylamino)-3-(2-oxo-1,2-dihydroquinolin-4-yl)propionate glutamate;
36) 2-morpholin-4-yl-ethyl 2-(4-chlorobenzoylamino)-3-(2-oxo-1,2-dihydroquinolin-4-yl)propionate glutarate;
37) 2-morpholin-4-yl-ethyl 2-(4-chlorobenzoylamino)-3-(2-oxo-1,2-dihydroquinolin-4-yl)propionate 2-oxoglutarate;
38) 2-morpholin-4-yl-ethyl 2-(4-chlorobenzoylamino)-3-(2-oxo-1,2-dihydroquinolin-4-yl)propionate glycerophosphorate ;
39) 2-morpholin-4-yl-ethyl 2-(4-chlorobenzoylamino)-3-(2-oxo-1,2-dihydroquinolin-4-yl)propionate hippurate;
40) 2-morpholin-4-yl-ethyl 2-(4-chlorobenzoylamino)-3-(2-oxo-1,2-dihydroquinolin-4-yl)propionate oleate;
41) 2-morpholin-4-yl-ethyl 2-(4-chlorobenzoylamino)-3-(2-oxo-1,2-dihydroquinolin-4-yl)propionate orotate;
42) 2-morpholin-4-yl-ethyl 2-(4-chlorobenzoylamino)-3-(2-oxo-1,2-dihydroquinolin-4-yl)propionate palmitate;
43) 2-morpholin-4-yl-ethyl 2-(4-chlorobenzoylamino)-3-(2-oxo-1,2-dihydroquinolin-4-yl)propionate pamoate;
44) 2-morpholin-4-yl-ethyl 2-(4-chlorobenzoylamino)-3-(2-oxo-1,2-dihydroquinolin-4-yl)propionate propionate;
45) 2-morpholin-4-yl-ethyl 2-(4-chlorobenzoylamino)-3-(2-oxo-1,2-dihydroquinolin-4-yl)propionate pyroglutamate;
46) 2-morpholin-4-yl-ethyl 2-(4-chlorobenzoylamino)-3-(2-oxo-1,2-dihydroquinolin-4-yl)propionate 4-aminosalicylate;
47) 2-morpholin-4-yl-ethyl 2-(4-chlorobenzoylamino)-3-(2-oxo-1,2-dihydroquinolin-4-yl)propionate sebacate;
48) 2-morpholin-4-yl-ethyl 2-(4-chlorobenzoylamino)-3-(2-oxo-1,2-dihydroquinolin-4-yl)propionate stearate;
49) 2-morpholin-4-yl-ethyl 2-(4-chlorobenzoylamino)-3-(2-oxo-1,2-dihydroquinolin-4-yl)propionate thiocyanate;
50) 2-morpholin-4-yl-ethyl 2-(4-chlorobenzoylamino)-3-(2-oxo-1,2-dihydroquinolin-4-yl)propionate undecylenate;
51) 2-morpholin-4-yl-ethyl 2-(4-chlorobenzoylamino)-3-(2-oxo-1,2-dihydroquinolin-4-yl)propionate hydrobromate;
52) 2-morpholin-4-yl-ethyl 2-(4-chlorobenzoylamino)-3-(2-oxo-1,2-dihydroquinolin-4-yl)propionate isobutyrate;
53) 2-morpholin-4-yl-ethyl 2-(4-chlorobenzoylamino)-3-(2-oxo-1,2-dihydroquinolin-4-yl)propionate laurate;
54) 2-morpholin-4-yl-ethyl 2-(4-chlorobenzoylamino)-3-(2-oxo-1,2-dihydroquinolin-4-yl)propionate DL-mandelate;
55) 2-morpholin-4-yl-ethyl 2-(4-chlorobenzoylamino)-3-(2-oxo-1,2-dihydroquinolin-4-yl)propionate methanesulfonate;
56) 2-morpholin-4-yl-ethyl 2-(4-chlorobenzoylamino)-3-(2-oxo-1,2-dihydroquinolin-4-yl)propionate naphthalene-1,5-disulfonate;
57) 2-morpholin-4-yl-ethyl 2-(4-chlorobenzoylamino)-3-(2-oxo-1,2-dihydroquinolin-4-yl)propionate naphthalene-2-sulfonate;
58) 2-morpholin-4-yl-ethyl 2-(4-chlorobenzoylamino)-3-(2-oxo-1,2-dihydroquinolin-4-yl)propionate 2-naphthoate;
59) 2-morpholin-4-yl-ethyl 2-(4-chlorobenzoylamino)-3-(2-oxo-1,2-dihydroquinolin-4-yl)propionate nicotinate;
60) 2-morpholin-4-yl-ethyl2-(4-chlorobenzoylamino)-3-(2-oxo-1,2-dihydroquinolin-4-yl)propionate nitrate;
61) 2-morpholin-4-yl-ethyl 2-(4-chlorobenzoylamino)-3-(2-oxo-1,2-dihydroquinolin-4-yl)propionate succinate;
62) 2-morpholin-4-yl-ethyl2-(4-chlorobenzoylamino)-3-(2-oxo-1,2-dihydroquinolin-4-yl)propionate sulfonate; and
63) 2-morpholin-4-yl-ethyl 2-(4-chlorobenzoylamino)-3-(2-oxo-1,2-dihydroquinolin-4-yl)propionate L-aspartate.

According to one embodiment of the present invention, the compound represented by Formula 1 may be represented by any one of the following Formulas 1-1 to 1-6:

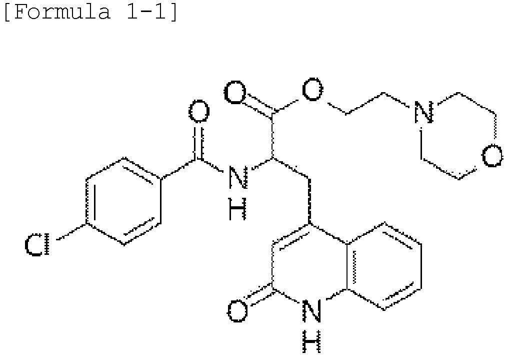

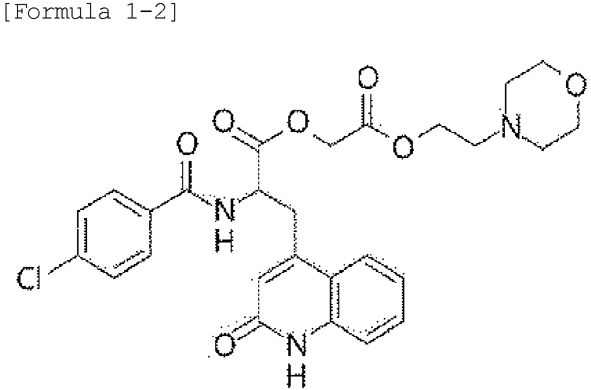

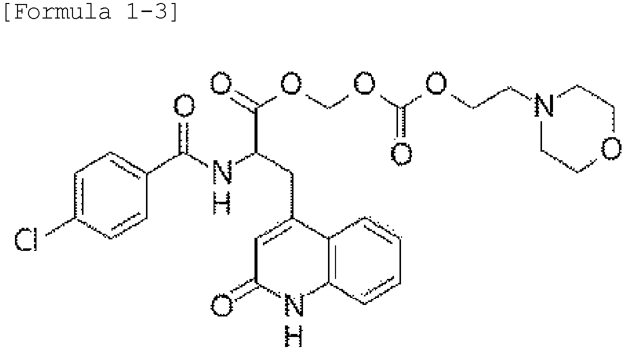

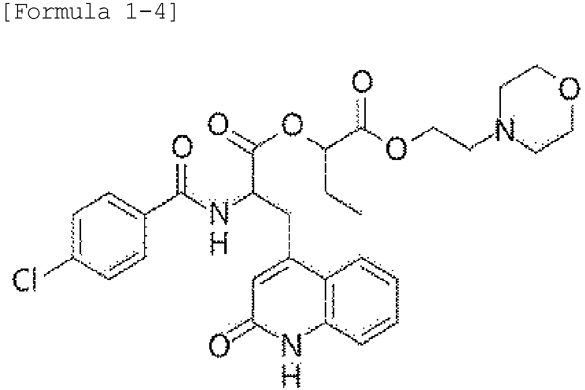

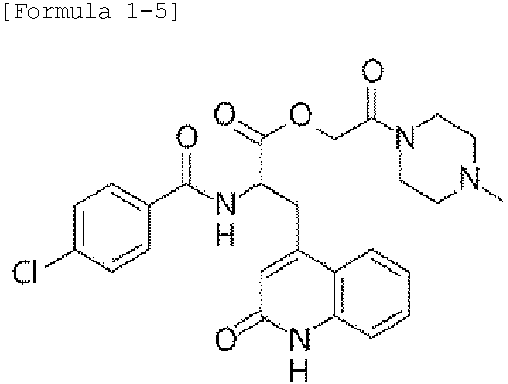

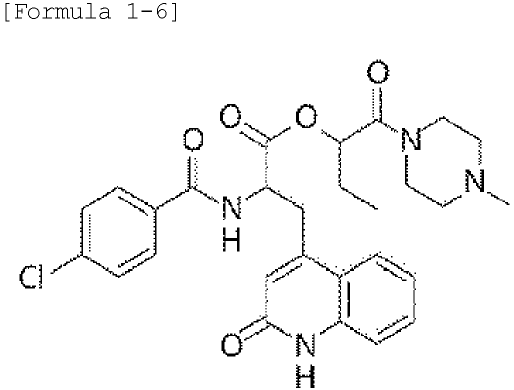

Meanwhile, a pharmaceutically acceptable salt of the compound of Formula 1 according to the present invention may be produced from the compound of Formula 1 produced by allowing a compound of the following Formula 2 to react with a compound of the following Formula 3: 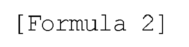 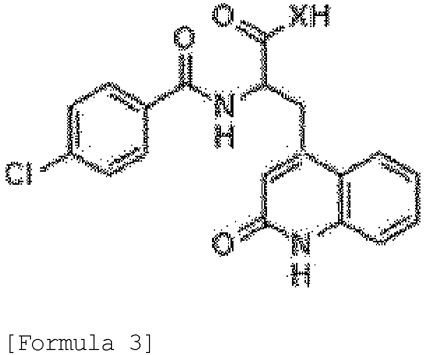 Y-Z
wherein X and Y are as defined above, and Z is hydroxy, amino, amine, a halogen or a leaving group.

In one embodiment of the present invention, Z is hydroxyl, -NH₂, Cl, Br, alkylsulfonyl or arylsulfonyl.

Specifically, a pharmaceutically acceptable salt of the compound of Formula 1 according to the present invention may be produced from the compound of Formula 1 produced by the method shown in the following Reaction Scheme 1, but is not limited thereto. 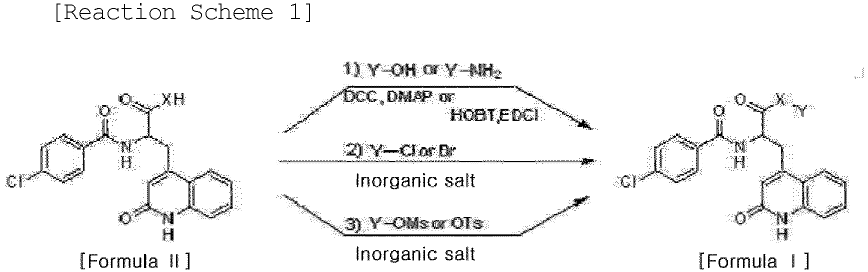 wherein X is any one of oxygen, nitrogen or sulfur.

The compound of Formula 2 used as the starting material in Reaction Scheme 1 above may be produced by the method described in US Patent No. 4,578,381. The inorganic salt shown in Reaction Scheme 1 above may be an inorganic base such as sodium bicarbonate, sodium carbonate, potassium bicarbonate, potassium carbonate or cesium carbonate, the solvent may be acetone, dimethylformamide, dimethyl sulfoxide, or acetonitrile, and the reaction may be carried out at 10 to 100 °C for 1 to 24 hours. In the above Reaction Scheme, DCC denotes dicyclohexylcarbodiimide; DMAP denotes 4-dimethylaminopyridine; HOBT denotes 1-hydroxybenzotriazole; and EDCI denotes 1-ethyl-3-(3-dimethylaminopropyl) carbodiimide hydrochloric acid. In addition, Y-OMs or Y-OTs is a sulfonyl group, and includes an alkylsulfonyl group such as a methanesulfonyl group; or an arylsulfonyl group such as paratoluenesulfonyl, benzenesulfonyl or 4-nitrobenzenesulfonyl group.

The compound of Formula 2 in which X in Reaction Scheme 1 above is sulfur may be produced in the process shown in Reaction Scheme 2 below.

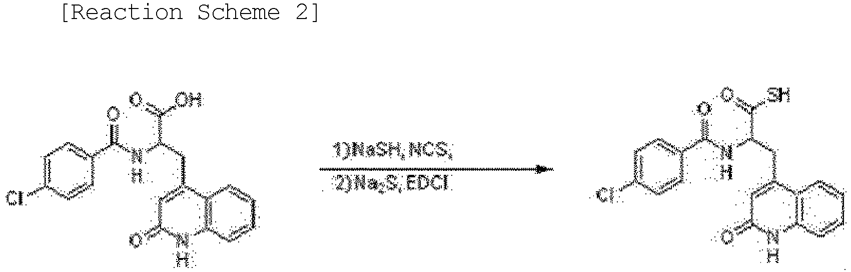

In Reaction Scheme 2 above, sodium hydrosulfide may be used in an amount of 1 to 10 equivalents, preferably 4 to 6 equivalents, and sodium sulfide may be used in an amount of 1 to 5 equivalents, preferably 2 to 3 equivalents. In Reaction Scheme 2 above, dimethylformamide, dimethyl sulfoxide or acetonitrile may be used as a solvent, and the reaction may be performed at 10 to 100°C for 1 to 24 hours. In the above reaction scheme, NCS refers to N-chlorosuccinimide.

A pharmaceutically effective amount of the compound represented by Formula 1 or a salt thereof according to the present invention may be 0.5 to 100 mg/day, specifically 1 to 30 mg/day per 1 kg of the patient's body weight. However, the pharmaceutically effective amount may be appropriately changed depending on the severity of disease symptoms, the patient's age, body weight, health condition and sex, the route of administration, and the period of treatment.

In addition, the composition according to the present invention may further contain pharmaceutically acceptable additives. As used herein, the term "pharmaceutically acceptable" means what is physiologically acceptable and, when administered to human beings, generally does not cause allergic reactions, such as gastrointestinal disorder and dizziness, or similar reactions thereto. Examples of the additives include carriers, excipients, and diluents. Examples of the carriers, excipients and diluents include lactose, dextrose, sucrose, sorbitol, mannitol, xylitol, erythritol, maltitol, starch, gum acacia, alginate, gelatin, calcium phosphate, calcium silicate, cellulose, methyl cellulose, polyvinylpyrrolidone, water, methylhydroxybenzoate, propylhydroxybenzoate, talc, magnesium stearate, and mineral oil. In addition, the composition may further contain a filler, an anti-aggregating agent, a lubricant, a wetting agent, a flavoring agent, an emulsifying agent, a preservative, and the like.

In addition, the pharmaceutical compositions of the present invention may be formulated using methods known in the art to provide rapid, sustained or delayed release of the active ingredient after administration to a mammal. In addition, the pharmaceutical composition of the present invention may be formulated so as to provide quick, sustained, or delayed release of the active ingredient after administration to a mammal by employing the methods known in the art. The formulation may be in the form of powders, granules, tablets, emulsions, syrups, aerosols, soft or hard gelatin capsules, sterile injectable solutions, or sterile powders.

The pharmaceutical composition according to the present invention may be administered through various routes including oral, transdermal, subcutaneous, intravenous or intramuscular routes. The dose of the active ingredient may be appropriately selected depending on various factors such as the route of administration, and the patient's age, sex, weight, and patient's disease severity. In addition, the active ingredient may be administered in combination with a compound known to have a preventive or therapeutic effect depending on the type of disease to be treated.

### [Mode for Invention]

Hereinafter, experiments for evaluating the anti-inflammatory effect and tissue regeneration effect of oral administration of the composition according to one embodiment of the present invention by using using a disease model of Sjogren's syndrome (*Nfkbiz*^{*-*/*-*} or IkB-**ξ**-deficient mice, C57BL/6) will be described in detail.

### 1. Preparation for Experiments

### 1.1 Experimental animals and Conditions

A 7-12-week old Sjogren's syndrome autoimmune disease model (*Nfkbiz*^{*-*/*-*} or IkB-**ξ**-deficient male mice, C57BL/6) was purchased from Seoul Asan Hospital. The environment of the animal laboratory was maintained under conditions of a temperature of 25±5°C and a humidity of 55 to 70% with a 12-hour light/12-hour dark cycle. The animals were allowed to access sterilized litter, pellet-type solid feed Teklad 2018S (Envigo, sterilizable) for laboratory animals, and sterile purified water *ad libitum.* Immediately after the animals were purchased, they were weighed and grouped in consideration of their age and Sjogren's syndrome phenotype. All animal experiments in this study were performed according to Standard Operation Procedures under the approval of the animal experiment ethics committee of Samjin Pharmaceutical Co., Ltd. (approval number: SJ-2016-001).

### 1.2 Experimental Tools and Reagents

- Experimental tools: sterilized cages, sterile water, a humidifier, a dehumidifier, a thermo-hygrostat, 1-cc syringes, markers, a balance, 20-ml vials, sondes (feeding needles), surgical tools, e-tubes, a Dissecting microscope, a clean bench, and micropipettes
- Reagents and materials: phenol red thread (Zone-Quick; Oasis Glendora, CA), iQ SYBR Green Supermix (Bio-rad), cDNA conversion kit (BR170-8891, Bio-rad), Alfaxalone (anesthetic), phospho-Akt (S473) antibody (Cell Signaling, #4060), IL-17A antibody (Santacruz, #sc-374218), beta-actin antibody (Sigma Aldrich, #A5316), pilocarpine hydrochloride (Sigma, P6503), heparin (Green Cross Corp.)
- RIPA lysis buffer (50 mM Tris-Cl pH7.4, 150 mM NaCl, 1 mM EDTA, 1% Triton X-100, 0.1% SDS, 1% sodium deoxicolate, 0.5 mM phenylmethylsulfonyl fluoride, 2 µg/ml leupeptin, 2 µg/ml aprotinin, 10 mM NaF, and 1mM sodium orthovanadate
- ELISA kit: IL-17A (Signosis, #EA-2516), anti-SSA (Signosis, #EA-5202), anti-SSB (Signosis, #EA-5204)

### 1.3. Experimental Groups and Excipient Composition

**[Table 1]**

| **Arms** | **Assigned Interventions** |
|---|---|
| Placebo Comparator (N/C, Ctrl) | Drug: Placebo (vehicle, 3% Tween80) |
| Active Comparator SA001 (80 mg/kg b.i.d) | Drug: SA001 oral administration 2 times per day for 5 weeks |

SA001 is 2-(morpholin-4-yl)ethyl 2-(4-chlorobenzoylamino)-3-(2-oxo-1,2-dihydroquinolin-4-yl)propionate malonate, and was produced by the following method.

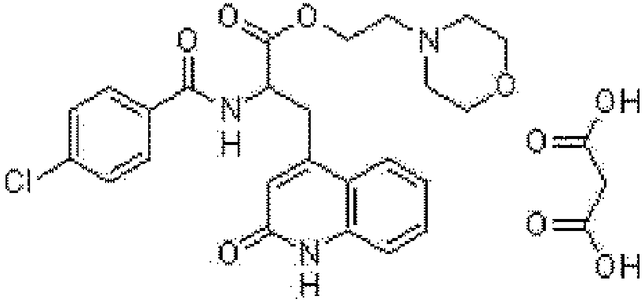

1 g (2.07 mmol) of 2-morpholin-4-ylethyl 2-(4-chlorobenzoylamino)-3-(2-oxo-1,2-dihydroquinolin-4-yl)propionate and 0.22 g (2.07 mmol) of malonic acid were reacted to obtain the title compound (1.1 g) as a white solid.
¹H NMR (400 MHz, DMSO-d6): 11.71 (s, 1H), 9.06 (d, 1H), 7.83 (t, 3H), 7.59 (s, 1H), 7.56 (s, 1H), 7.52 (t, 1H), 7.32 (d, 1H), 7.24 (t, 1H), 6.46 (s, 1H), 4.77 (m, 1H), 4.24 (m, 2H), 3.51-3.46 (m, 5H), 3.29 (q, 1H), 3.19 (s, 2H), 2.70-2.61 (m, 2H), 2.51 (t, 2H), 2.47 (br-s, 4H)

### 2. Experimental Method

### 2.1 Administration Mode and Administration Duration : Oral administration (P.O), twice a day (10 h, 16 h), for 5 weeks (35 days)

### 2.2 Anesthesia (I.V) : Alfaxalone (13 mg/kg ; PBS diluted)

### 2.3 Tear Production Measurement (Schirmer test)

Tear production was measured using a phenol red thread (Zone-Quick; Oasis, Glendora, CA) 1 minute after animal anesthesia. One end of the phenol red thread was placed against the lower eyelid of the lateral canthus of the animal eye and left to stand for 60 seconds. After the thread was soaked with tear by the capillary phenomenon, the length (mm) of the thread whose color changed was measured. When the amount of tear secretion decreases, dry eye caused by Sjogren syndrome symptoms is promoted. Thus, it was determined that the greater the amount of tear secretion, the greater the effect of the test substance.

### 2.4 Measurement of Salivary Flow Rate

Pilocarpine hydrochloride (Sigma, P6503, stored at 4°C after making a concentration of 1 mg/ml in PBS) was injected intraperitoneally (I.P. injection) in an amount of 100 µl/animal, and after 2 minutes, the animals were anesthetized and the salivary flow rate was measured by collecting saliva with a micropipette.

### * Saliva flow rate in µL / minute = saliva (µL) / 3

### 2.5 Isolation of Lacrimal Gland and Salivary Gland Tissues and Quantification of Protein Expression (Immunoblotting Analysis)

The lacrimal gland and salivary gland tissues of the test animals were isolated, placed in PBS, and homogenized on ice. 1 ml of RIPA lysis buffer (50 mM Tris-Cl pH7.4, 150 mM NaCl, 1 mM EDTA, 1% Triton X-100, 0.1% SDS, 1% sodium deoxycholate, 0.5 mM phenylmethylsulfonyl fluoride, 2 µg/ml leupeptin, 2 µg/ml aprotinin, 10 mM NaF, and 1 mM sodium orthovanadate) was added to each homogenized tissue which was then kept on ice for 5 minutes. Each tissue solution was then centrifuged (at 13000 rpm for 20 mins), and only the supernatant was collected, and then protein correction was performed. Samples were incubated with anti-Akt (1:400) and anti-interleukin 17A (1:200) antibodies and subjected to immunoprecipitation (IP) (overnight), followed by sampling. Each of the samples was subjected to SDS-PAGE and transferred to polyvinylidene fluoride (PVDF) membranes. Then, the Akt-IP-samples were incubated with phospho-Akt (S473) (diluted at 1:100,000 in blocking buffer (TBS + Tween20) + 0.1% skim milk) at 4°C overnight, and the IL-17A-IP-samples were incubated with IL-17A primary antibody (diluted at 1:100,000 in blocking buffer (TBS + Tween20) + 0.1% skim milk) at 4°C overnight. Next, the samples were incubated with HRP-conjugated secondary antibody, and then protein bands were visualized with ECL solution in the dark and quantified by exposure to X-ray films.

### 2.6 Isolation and Quantification (Quantitative RT-PCR) of Total mRNA from Lacrimal Gland and Salivary Gland Tissues

From half of each tissue homogenized with PBS, total RNA was isolated using TRIzol according to manufacturer's protocols and converted into cDNA using a reverse transcription system (iScript cDNA synthesis BR170-8891, Bio-Rad, U.S). From the cDNA, mRNA was quantified using real-time PCR. The primers used in the PCR were synthesized by Bionics Co Ltd. (Seoul, Korea). The primers used in the PCR were as follows: mouse cytokines: *IL-2 (forward 5'-TTG AGT GCC AAT TCG ATG AT-3' and reverse 5'-TTG AGG GCT TGT TGA GAT GA-3'); IL-17A (forward 5 '-TGT CTC TGA TGC TGT TGC TG-3' and reverse 5'- AGT CCT TGG CCT CAG TGT TT-3'); IFN-g (forward 5'-AGC TCT TCC TCA TGG CTG TT-3' and reverse 5'-TTT GCC AGT TCC TCC AGA TA-3'); BAFF (forward 5'-GGT GAC CCT GTT CCG ATG TA-3' and reverse 5'- CTC CGT TGC GTG AAA TCT GT-3'); FLT3L (forward 5'-TGG CAG GGT CTA AGA TGC AA-3' and reverse 5'-AGG TGG GAG ATG TTG GTC TG-3'); IL-1 (forward 5'-AGC TTC AAA TCT CGC AGC AG-3' and reverse 5'-TCT CCA CAG CCA CAA TGA GT-3'); IL-6 (forward 5'-GAC TGA TGC TGG TGA CAA CC-3' and reverse 5'-AGA CAG GTC TGT TGG GAG TG-3'); IL-12A (forward 5'-CCT GCA CTG CTG AAG ACA TC-3' and reverse 5'-CCA GGC AAC TCT CGT TCT TG-3'); IL-18 (forward 5'-GCT GCC ATG TCA GAA GAC TC-3' and reverse 5'-ACT GCG GTT GTA CAG TGA AG-3'); IL-21 (forward 5'-AGT GGC CCA TAA ATC AAG CC -3' and reverse 5'-AAA GCT GCA TGC TCA CAG T-3'), IL-22 (forward 5'-AAT CAG CTC AGC TCC TGT CA-3' and reverse 5'-TCG CCT TGA TCT CTC CAC TC-3'); TNF-(forward 5'-TCT CTT CAA GGG ACA AGG CT-3' and reverse 5'-GGC AGA GAG GAG GTT GAC TT-3'); IFN- (forward 5'-GCT CTG TGC TTT CCT GAT GG-3' and reverse 5'-GTA CCA GGA GTG TCA AGG CT-3'); chemokines: CXCL13 (forward 5'-ATG AGG CTC AGC ACA GCA A-3' and reverse 5'-GAA TAC CGT GGC CTG GAG AG-3'); CXCR5 (forward 5'-CTC AAC CGA GAC CTT CCT GT-3' and reverse 5'-GTG CAG AGC GAT CAC AGT TT-3'); and GAPDH (forward 5'- TGG AGA AAC CTG TAT GTA TGG G-3' and reverse 5'- GGC CTG ATA AGA ACA AAC CC-3').* The PCR was performed by reacting a mixture of SsoAdvancedT^{M} Universal SYBR Green Supermix (Bio-rad), each primer (0.5 pmole) and template cDNA using a PCR system (CFX connect, Bio-Rad, U.S) under the following conditions: initial denaturation at 95 °C for 3 min; 39 cycles, each consisting of denaturation at 95°C 10 for sec, annealing at 55°C for 30 sec, and extension at 65°C for 5sec.

### 2.7 Quantification (ELISA) of Autoantibodies and cytokine (IL-17A) in Plasma

As markers of Sjogren's syndrome, anti-SSA/Ro (Signosis, #EA-5202, US) and anti-SSB/La (Signosis, #EA-5204, US), which are autoantibodies based on RNP particles, and interleukin-17A cytokine (Signosis, #EA-2516, US) which is involved in the onset of autoimmune diseases, were quantified by Enzyme-linked ImmunoSorbent Assay (ELISA) in order to determine the secretion and production of these markers in serum.

### 2.8 Analysis of Inflammatory Phenotypes in Animals (Disease Scores)

The degree of inflammation in the animal model was scored according to the following criteria and analyzed.
1: periorbital swelling
2: eyes with discharge that were difficult to open
3: periocular skin erosion
4: inflammation reaching perioral skin
5: erosion and loss of hair in whole face.

### 2.9 Statistics

The experimental results were expressed as mean ± standard error (SE) and analyzed through Dunnett's t test after one-way analysis of variance (ANOVA) using IBM SPSS Statics 21 (IBM Corporation, Poughkeepsie, NY, USA). Probability (p)-value <0.05 was considered statistically significant.

### 3. Experimental Results

### 3.1 Effect on Tear Production

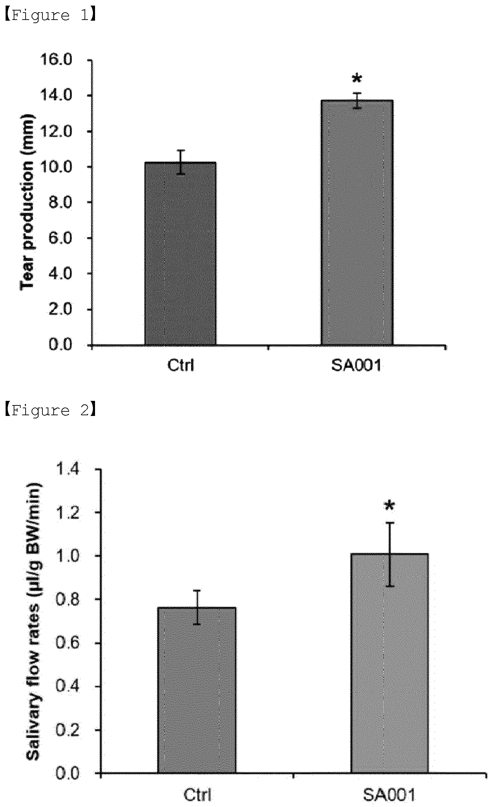

FIG. 1 is a graph showing tear production in the disease model of Sjogren's syndrome. Referring to FIG. 1, it can be confirmed that tear production in the disease model of Sjogren's syndrome is significantly increased by administration of the composition according to the present invention (mean ± SE., N=11). *p<0.05 versus the control (Ctrl) group.

### 2.2 Effect on Salivary Flow Rate

FIG. 2 is a graph showing salivary flow rate in the disease model of Sjogren's syndrome. Referring to FIG. 2, it could be confirmed that the salivary flow rate (1.00.2) in the group to which the composition according to the present invention was administered significantly increased compared to that in the control group (0.750.05) (mean ± SE., N=11). *p<0.05 versus the control (Ctrl) group.

### 2.3 Effect on Protein Expression of Cytokine (IL-17A) and Phospho-Akt in Salivary and Lacrimal Glands

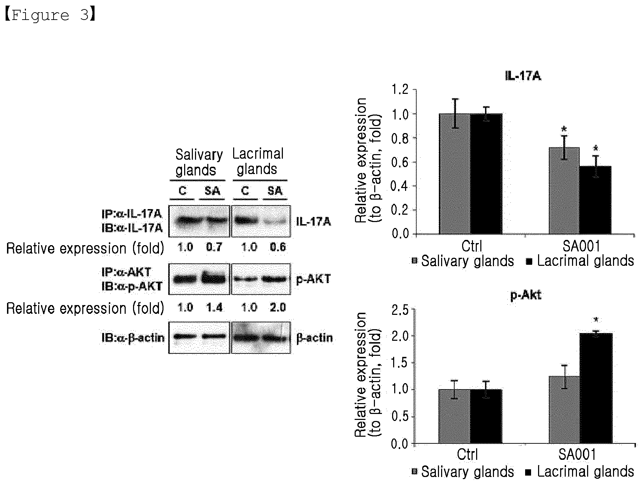

FIG. 3 shows the results of measuring the protein expressions of IL-17A and p-AKT and the results of quantifying the same.

Autoimmune diseases are mainly caused by differentiation and activation of helper 17 T cells (Th17), and the severity of autoimmune diseases may be evaluated by quantifying increased expression of the pro-inflammatory cytokine interleukin-17A (IL-17A) produced by these T cells.

As a result of quantifying the protein expression of IL-17A in the lacrimal gland and salivary gland tissues of the group to which the composition according to the present invention was administered at a dose of 100 mg/kg, it was confirmed that the protein expression of IL-17A in the group significantly decreased compared to that in the control group.

In addition, as a result of quantifying p-AKT, an activated form of Akt involved in cell survival and growth, it was confirmed that the expression of p-AKT significantly increased in the group to which the composition according to the present invention was administered (mean ± SE., N=3). *p<0.05 versus the control (Ctrl) group. Therefore, it can be seen that the composition according to the present invention has an inflammation-alleviating effect and a tissue regeneration effect.

### 2.4 Effect on mRNA Expression of Various Pro-Inflammatory and Chemotactic Cytokines in Salivary and Lacrimal Glands

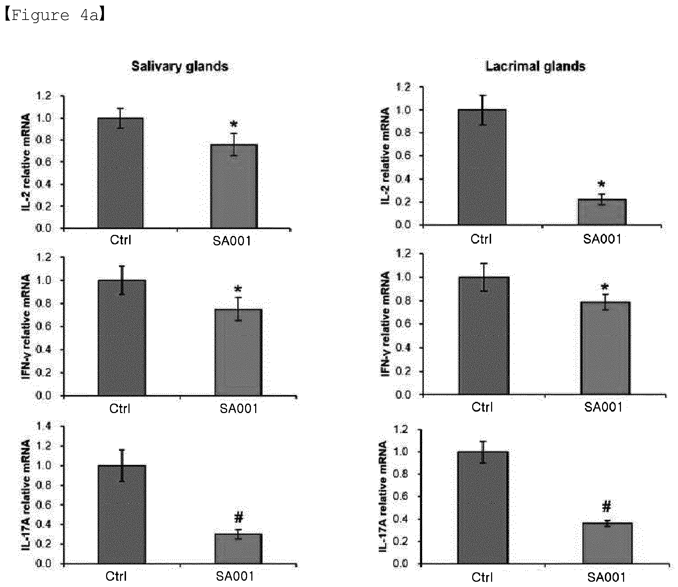

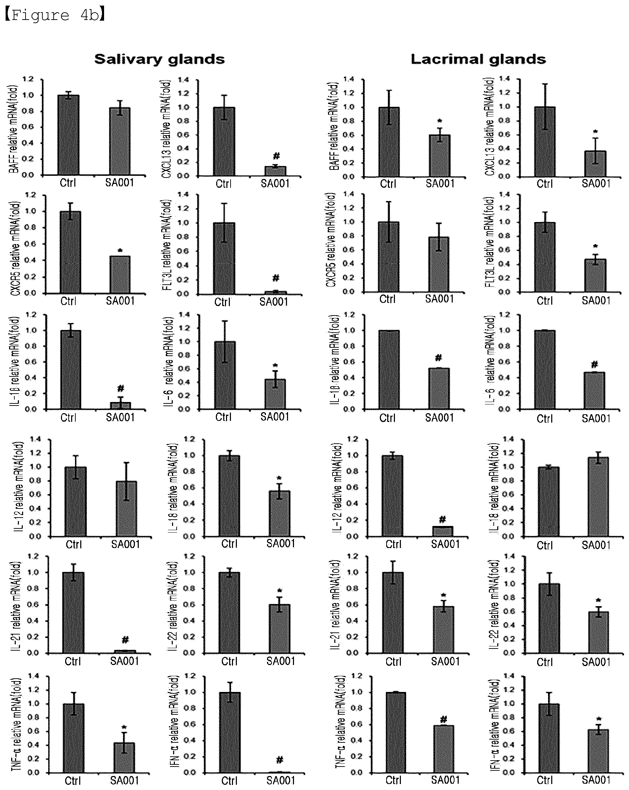

FIGS. 4a and 4b show the results of quantifying the mRNAs of pro-inflammatory cytokines and chemotactic cytokines.

The results of comparing the results of quantification of the mRNAs of autoimmune disease-related immune cells (T cells and B lymphocytes)-derived pro-inflammatory and chemotactic cytokines in the lacrimal gland and salivary gland tissues with the results obtained in the control are as follows:
IL-2 (salivary glands: 1.3-fold, lacrimal glands: 4.5-fold); interferon-g (salivary glands: 1.3-fold, lacrimal glands: 1.3-fold); IL-17A (salivary glands: 3.3-fold, lacrimal glands: 2.8-fold); BAFF (salivary glands: not significant, lacrimal glands: 1.6-fold); CXCL13 (salivary glands: 5-fold, lacrimal glands: 2.5-fold); CXCR5 (salivary glands: 2.5-fold, lacrimal glands: not significant); FLT3L (salivary glands: 13-fold, lacrimal glands: 2.5-fold); IL-1β (salivary glands: 10-fold, lacrimal glands: 2-fold); IL-6 (salivary glands: 2.5-fold, lacrimal glands: 2-fold); IL-12 (salivary glands: not significant, lacrimal glands: 10-fold); IL-18 (salivary glands: 1.6-fold, lacrimal glands: not significant); IL-21 (salivary glands: 20-fold, lacrimal glands: 1.6-fold); IL-22 (salivary glands: 1.6-fold, lacrimal glands: 1.7-fold); TNF-α (salivary glands: 2.2-fold, lacrimal glands: 1.6-fold); and IFN-α (salivary glands: 40-fold, lacrimal glands: 1.6-fold). Thus, it could be seen that most of the cytokines were significantly decreased by administration of the composition according to the present invention (mean ± SE., N=6). *p<0.05, #p<0.01 versus the control (Ctrl) group.

### 3.5 Effect on Changes in Secretion of Autoantibodies (Anti-SSA/Ro, and Anti-SSB/La) and Cytokine (IL-17A) in Plasma of Disease Animal Model

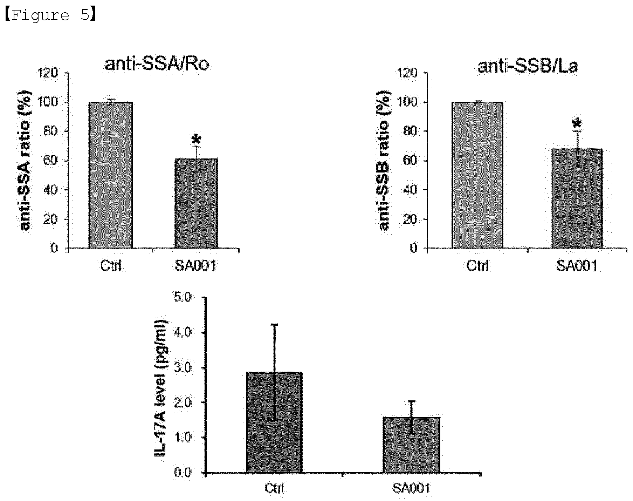

FIG. 5 shows the results of measuring the protein expressions of IL-17A and autoantibodies (anti-SSA/Ro, and anti-SSB/La) in plasma.

As Sjogren's syndrome develops, the *in vivo* autoantigens SSA/Ro and SSB/La increase. Thereby, the B cells of the immune system produce autoantibodies. By measuring the presence or absence of the autoantibodies (anti-SSA/Ro, and anti-SSB/La), it is possible to evaluate the severity of Sjogren's syndrome.

Referring to Table 2 below and FIG. 5, it can be seen that, in the group to which the composition (SA001) according to the present invention was administered, both anti-SSA (40%) and anti-SSB (30%) in plasma significantly decreased compared to those in the control group (see Table 1), and IL-17A also decreased (mean ± SE., N=3). *p<0.05 versus the control (Ctrl) group.

**[Table 2]**

| | IkB-ξ-deficient male mice, | |
|---|---|---|
| | No.(%) vehiclen=3 | No. (%) SA001(80mg/kg)n=3 |
| SSA/Ro | 100.0±2.0 | 61.0±8.7 |
| SSB/La | 100.0±0.9 | 67.9±12.4 |

### 3.6 Changes in Inflammatory Phenotypes in Disease Animal Model

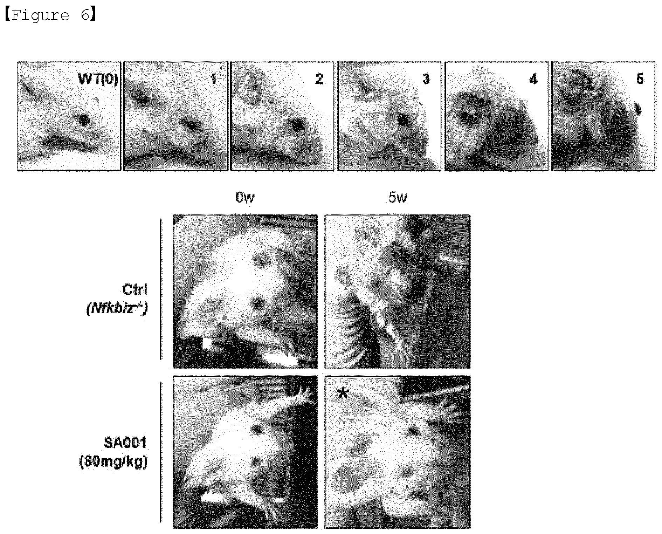

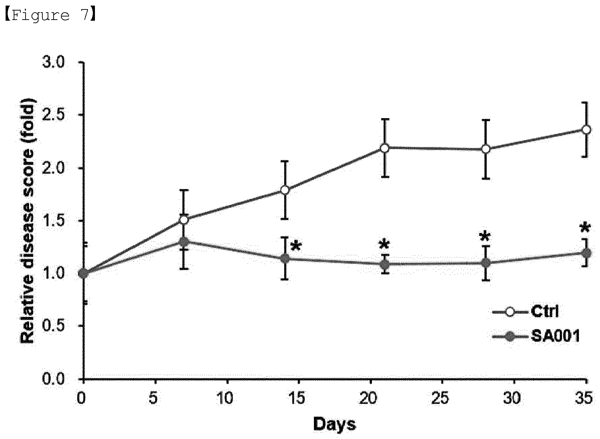

FIG. 6 depicts photographs showing changes in the inflammatory phenotypes in the disease animal model, and FIG. 7 shows disease scores obtained by scoring the phenotypic changes.

As Sjogren's syndrome develops, keratin increases apparently, and inflammation occurs due to severe dryness of the eyeball and drooling symptoms. Thus, as shown in FIG. 6, the inflammatory phenotype was evaluated on a five-point scale (disease score). A higher score indicates a higher degree of inflammation. Referring to FIG. 7, it was observed that the onset of the disease in the SA001-administered group was significantly slowed compared to that in the control group from 2 weeks after administration (mean ± SE., N=11). *p<0.05 versus the control (Ctrl) group.

As described above, tear production, salivary flow rate, inflammatory cytokines in various tissues (lacrimal glands and salivary glands), and autoantibodies (anti-SSA/Ro and anti-SSB/La) and IL-17A in plasma were compared between the groups.

Tear production and salivary flow rate tended to increase significantly in the group to which the composition of the present invention was administered. The mRNA expression levels of immune cell-derived pro-inflammatory cytokines and chemotactic cytokines in the lacrimal gland and salivary gland tissues significantly decreased in the drug-administered group. In addition, it was confirmed that the composition of the present invention decreased the expression level of IL-17A cytokine protein in the lacrimal gland and salivary gland tissues and increased the level of activated Akt, which indicates the survival and growth of cells constituting the tissues, suggesting that the composition of the present invention may alleviate inflammation and regenerates tissues. It was observed that, in the group to which the composition of the present invention was administered, autoantibodies (anti-SSA, anti-SSB) (which are markers of Sjogren's syndrome in plasma, which increase with the onset of Sjogren's syndrome) significantly decreased and the secretion of IL-17A cytokine also decreased. As a result of comparing the degree of inflammation through observation of the apparent phenotype, it was confirmed that administration of the composition of the present invention has the effect of slowing the onset of the disease. That is, it has been confirmed that administration of the composition of the present invention may slow the onset of the disease in the disease model, may relieve inflammation in the directly related lacrimal and salivary gland tissues, and may increase the growth of cells constituting the tissues. Therefore, it is concluded that the composition of the present invention is effective in preventing and treating Sjogren's syndrome.

The above description of the present invention is exemplary, and those of ordinary skill in the art will appreciate that the present invention can be easily modified into other specific forms without departing from the technical spirit or essential characteristics of the present invention. Therefore, it should be understood that the exemplary embodiments described above are exemplary in all aspects and are not restrictive. For example, each component described to be of a single type can be implemented in a distributed manner. Likewise, components described to be distributed can be implemented in a combined manner.

The scope of the present invention is defined by the following claims rather than by the detailed description of the invention. It shall be understood that all modifications and embodiments conceived from the meaning and scope of the claims and the equivalents thereto are included in the scope of the present invention.

## Claims

1. A composition for preventing or treating Sjogren's syndrome, the composition containing a compound represented by the following Formula 1 or a pharmaceutically acceptable salt thereof. 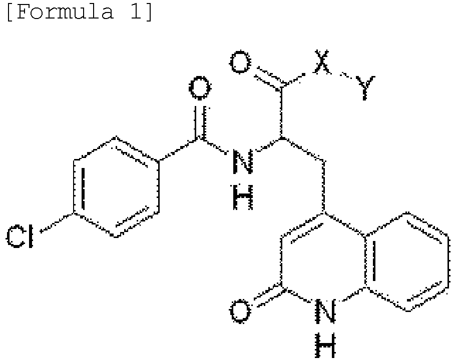 wherein
X is any one of oxygen, nitrogen or sulfur; and
Y is C₁-C₆ alkyl, C₁-C₆ haloalkyl, (C₁-C₃ alkyloxy) C₁-C₆ alkyl, (C₂-C₆ alkenyloxy) C₁-C₆ alkyl, (C₁-C₆ alkylcarbonyloxy) C₁-C₆ alkyl, (C₁-C₆ alkylsulfanyl) C₁-C₆ alkyl, (arylsulfanyl) C₁-C₆ alkyl, (arylsulfonyl) C₁-C₆ alkyl, (C₁-C₆ alkylamino) C₁-C₆ alkyl, [(C₁-C₆ alkyl) (C₁-C₆ alkyl) amino] C₁-C₆ alkyl, [(C₁-C₃ alkyl) (aryl) amino] C₁-C₆ alkyl, [(C₁-C₃ alkyl) (aryl) C₁-C₃ alkyl]amino C₁-C₆ alkyl, [(C₁-C₃ alkyl)(heteroaryl)amino]C₁-C₆ alkyl, (arylcarbonylamino)C₁-C₆ alkyl, C₂-C₆ alkenyl, C₂-C₆ alkynyl, C₂-C₆ oxoalkyl, C₃-C₈ cycloalkyl, (C₃-C₈ cycloalkyl) C₁-C₆ alkyl, (C₃-C₈ cycloalkenyl) C₁-C₆ alkyl, (C₃-C₈ heterocycloalkyl) C₁-C₆ alkyl, [(C₁-C₃ alkyl) C₃-C₈ heterocycloalkyl]C₁-C₆ alkyl, [(aryl)C₁-C₃ alkyl] C₃-C₈ heterocycloalkylC₁-C₆ alkyl, [(C₁-C₆ alkyloxycarbonyl)C₃-C₈ heterocycloalkyl]C₁-C₆ alkyl, [(C₁-C₃ alkyloxycarbonyl)C₃-C₈ heterocycloalkyl] C₁-C₆ alkyl, (C₃-C₈ heterocycloalkyl) C₁-C₆ alkenyl, [(C₁-C₃ alkyl) C₃-C₈ heterocycloalkenyl]C₁-C₆ alkyl, (aryl) C₁-C₆ alkyl, [(C₁-C₃ alkyl) aryl] C₁-C₆ alkyl, [(C₁-C₃ alkyloxy) aryl] C₁-C₆ alkyl, [(aryloxy)aryl]C₁-C₆ alkyl, [(C₁-C₃ alkylsulfanyl)aryl]C₁-C₆ alkyl, [(C₁-C₃ alkyloxycarbonyl)aryl]C₁-C₆ alkyl, [(aryloxycarbonyl) aryl]C₁-C₆ alkyl, (aryl)C₃-C₆ alkenyl, (heteroaryl)C₁-C₆ alkyl, [(alkyloxycarbonyl)heteroaryl]C₁-C₆ alkyl, [(C₁-C₃ alkyl) C₃-C₈ heteroaryl]C₁-C₆ alkyl, [(C₃-C₈ cycloalkyl)heteroaryl]C₁-C₆ alkyl, [(aryl)heteroaryl]C₁-C₆ alkyl, [(C₁-C₃ alkyl) heteroaryl]C₁-C₆ alkyl, [(aryl) C₁-C₃ alkyl]heteroaryl C₁-C₆ alkyl, (C₁-C₆ alkyloxycarbonyl) C₁-C₆ alkyl, [(C₃-C₈ heterocycloalkyl) C₁-C₆ alkyloxycarbonyl]C₁-C₆ alkyl, (C₃-C₈ heterocycloalkylcarbonyl)C₁-C₆ alkyl, [(C₁-C₃ alkyl)C₃-C₈ heterocycloalkylcarbonyl]C₁-C₆ alkyl, [(C₁-C₃ alkyl)C₃-C₈ heterocycloalkylcarbonyl]C₁-C₆ alkyl, [(C₃-C₈ cycloalkyl) oxycarbonyloxy]C₁-C₆ alkyl, [(C₃-C₈ heterocycloalkyl)oxycarbonyloxy]C₁-C₆ alkyl, (ureido)C₁-C₆ alkyl, (arylureido) C₁-C₆ alkyl, [(aryl) (C₁-C₃alkylureido]C₁-C₆ alkyl, (C₁-C₆ alkylaminocarbonyl) C₁-C₆ alkyl, [(C₃-C₈ heterocycloalkyl)aminocarbonyl]C₁-C₆ alkyl, [(C₁-C₃ alkyl)C₃-C₈ heterocycloalkyl]aminocarbonyl C₁-C₆ alkyl, [(C₁-C₃ alkyl) (C₁-C₃ alkyloxy) aminocarbonyl] C₁-C₆ alkyl, or (oxo C₃-C₈ heterocycloalkyl) C₁-C₆ alkyl,
wherein the C₁-C₆ alkyl, C₂-C₆ alkenyl, C₂-C₆ alkynyl, C₂-C₆ oxoalkyl, C₃-C₈ cycloalkyl, C₃-C₈ cycloalkenyl, C₃-C₈ heterocycloalkenyl, aryl or heteroaryl may be substituted with at least one substituent selected from the group consisting of C₁-C₃ alkyl, fluoro, chloro, bromo, hydroxy, oxo, nitro and cyano groups.

2. The composition of claim 1, wherein the compound represented by Formula 1 is represented by any one of the following Formulas 1-1 to 1-6:

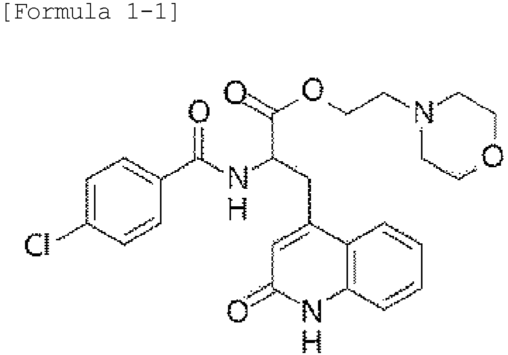

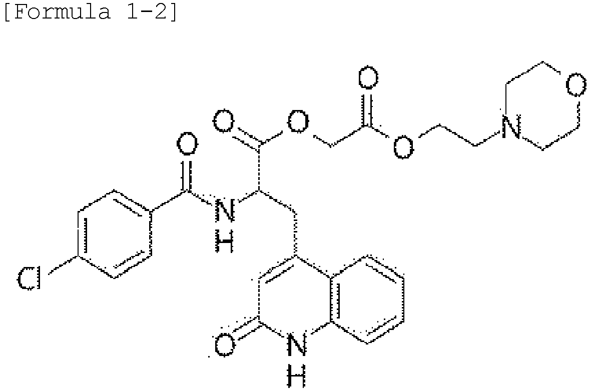

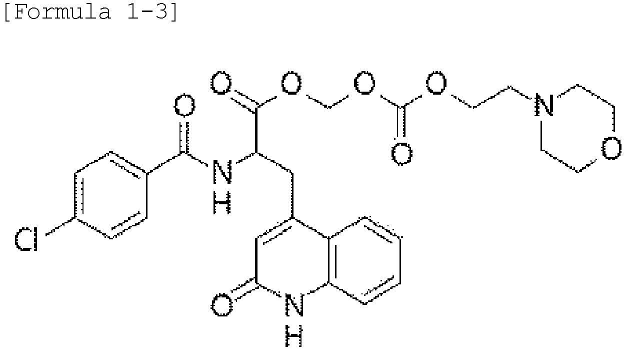

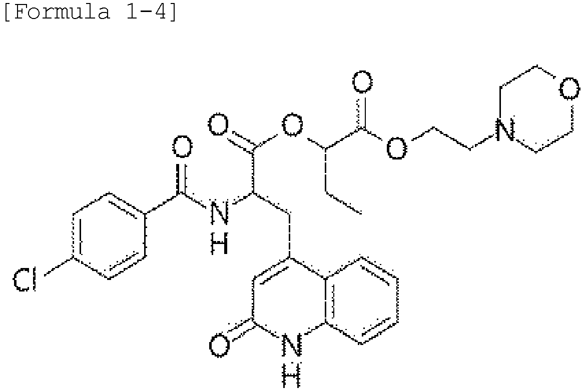

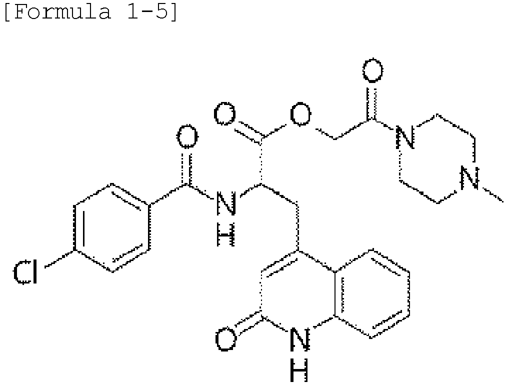

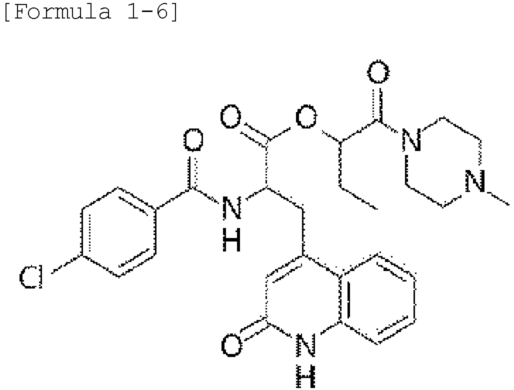

3. The composition of claim 1, wherein the salt is an acid addition salt formed by a pharmaceutically acceptable free acid.

4. The composition of claim 3, wherein the free acid is an organic acid or an inorganic acid.

5. The composition of claim 4, wherein the organic acid is selected from the group consisting of citric acid, acetic acid, lactic acid, tartaric acid, maleic acid, fumaric acid, formic acid, propionic acid, oxalic acid, dichloroacetic acid, trifluoroacetic acid, adipic acid, ascorbic acid, benzoic acid, 4-acetamidobenzoic acid, gluconic acid, sulfonic acid, methanesulfonic acid, capric acid, capronic acid, caprylic acid, carboxylic acid, cinnamic acid, cyclamic acid, dodecylsulfonic acid, ethane-1,2-disulfonic acid, ethanesulfonic acid, 2-hydroxyethanesulfonic acid, galactaric acid, gentisic acid, glutaric acid, 2-oxoglutaric acid, glycerophosphoric acid, hippuric acid, oleic acid, orotic acid, palmitic acid, pamoic acid, pyroglutamic acid, sebacic acid, stearic acid, thiocyanic acid, undecylenic acid, isobutyric acid, lauric acid, mandelic acid, naphthalene-1,5-disulfonic acid, naphthalene-2-sulfonic acid, naphthoic acid, nicotinic acid, glycolic acid, succinic acid, 4-toluenesulfonic acid, camphorsulfonic acid, glutamic acid, aspartic acid, salicylic acid, 4-aminosalicylic acid, malonic acid, malic acid, and benzosulfonic acid, and
the inorganic acid is selected from the group consisting of hydrochloric acid, bromic acid, sulfuric acid, nitric acid, and phosphoric acid.

6. The composition of claim 1, wherein the compound of Formula 1 is selected from the following:
1) 2-(morpholin-4-yl)ethyl 2-(4-chlorobenzoylamino)-3-(2-oxo-1,2-dihydroquinolin-4-yl)propionate glycolate;
2) 2-(morpholin-4-yl)ethyl 2-(4-chlorobenzoylamino)-3-(2-oxo-1,2-dihydroquinolin-4-yl)propionate lactate;
3) 2-(morpholin-4-yl)ethyl 2-(4-chlorobenzoylamino)-3-(2-oxo-1,2-dihydroquinolin-4-yl)propionate salicylate;
4) 2-(morpholin-4-yl)ethyl 2-(4-chlorobenzoylamino)-3-(2-oxo-1,2-dihydroquinolin-4-yl)propionate oxalate;
5) 2-(morpholin-4-yl)ethyl 2-(4-chlorobenzoylamino)-3-(2-oxo-1,2-dihydroquinolin-4-yl)propionate malonate;
6) 2-(morpholin-4-yl)ethyl 2-(4-chlorobenzoylamino)-3-(2-oxo-1,2-dihydroquinolin-4-yl)propionate maleate;
7) 2-(morpholin-4-yl)ethyl 2-(4-chlorobenzoylamino)-3-(2-oxo-1,2-dihydroquinolin-4-yl)propionate tartrate;
8) 2-(morpholin-4-yl)ethyl 2-(4-chlorobenzoylamino)-3-(2-oxo-1,2-dihydroquinolin-4-yl)propionate maleate;
9) 2-(morpholin-4-yl)ethyl 2-(4-chlorobenzoylamino)-3-(2-oxo-1,2-dihydroquinolin-4-yl)propionate fumarate;
10) 2-(morpholin-4-yl)ethyl 2-(4-chlorobenzoylamino)-3-(2-oxo-1,2-dihydroquinolin-4-yl)propionate citrate;
11) 2-(morpholin-4-yl)ethyl 2-(4-chlorobenzoylamino)-3-(2-oxo-1,2-dihydroquinolin-4-yl)propionate benzenesulfonate;
12) 2-(morpholin-4-yl)ethyl 2-(4-chlorobenzoylamino)-3-(2-oxo-1,2-dihydroquinolin-4-yl)propionate tosylate;
13) 2-(morpholin-4-yl)ethyl 2-(4-chlorobenzoylamino)-3-(2-oxo-1,2-dihydroquinolin-4-yl)propionate hydrochloride;
14) 2-(morpholin-4-yl)ethyl 2-(4-chlorobenzoylamino)-3-(2-oxo-1,2-dihydroquinolin-4-yl)propionate sulfate;
15) 2-(morpholin-4-yl)ethyl 2-(4-chlorobenzoylamino)-3-(2-oxo-1,2-dihydroquinolin-4-yl)propionate phosphrate;
16) 2-morpholin-4-yl-ethyl 2-(4-chlorobenzoylamino)-3-(2-oxo-1,2-dihydroquinolin-4-yl)propionate acetate;
17) 2-morpholin-4-yl-ethyl 2-(4-chlorobenzoylamino)-3-(2-oxo-1,2-dihydroquinolin-4-yl)propionate dichloroacetate;
18) 2-morpholin-4-yl-ethyl 2-(4-chlorobenzoylamino)-3-(2-oxo-1,2-dihydroquinolin-4-yl)propionate adipate;
19) 2-morpholin-4-yl-ethyl 2-(4-chlorobenzoylamino)-3-(2-oxo-1,2-dihydroquinolin-4-yl)propionate ascorbate;
20) 2-morpholin-4-yl-ethyl 2-(4-chlorobenzoylamino)-3-(2-oxo-1,2-dihydroquinolin-4-yl)propionate benzoate;
21) 2-morpholin-4-yl-ethyl 2-(4-chlorobenzoylamino)-3-(2-oxo-1,2-dihydroquinolin-4-yl)propionate 4-acetamidobenzoate;
22) 2-morpholin-4-yl-ethyl 2-(4-chlorobenzoylamino)-3-(2-oxo-1,2-dihydroquinolin-4-yl)propionate caprate;
23) 2-morpholin-4-yl-ethyl 2-(4-chlorobenzoylamino)-3-(2-oxo-1,2-dihydroquinolin-4-yl)propionate capronate;
24) 2-morpholin-4-yl-ethyl 2-(4-chlorobenzoylamino)-3-(2-oxo-1,2-dihydroquinolin-4-yl)propionate caprylate;
25) 2-morpholin-4-yl-ethyl 2-(4-chlorobenzoylamino)-3-(2-oxo-1,2-dihydroquinolin-4-yl)propionate carbonate;
26) 2-morpholin-4-yl-ethyl 2-(4-chlorobenzoylamino)-3-(2-oxo-1,2-dihydroquinolin-4-yl)propionate cinnamate;
27) 2-morpholin-4-yl-ethyl 2-(4-chlorobenzoylamino)-3-(2-oxo-1,2-dihydroquinolin-4-yl)propionate cyclamate;
28) 2-morpholin-4-yl-ethyl 2-(4-chlorobenzoylamino)-3-(2-oxo-1,2-dihydroquinolin-4-yl)propionate dodecylsulfonate;
29) 2-morpholin-4-yl-ethyl 2-(4-chlorobenzoylamino)-3-(2-oxo-1,2-dihydroquinolin-4-yl)propionate ethane-1,2-disulfonate;
30) 2-morpholin-4-yl-ethyl 2-(4-chlorobenzoylamino)-3-(2-oxo-1,2-dihydroquinolin-4-yl)propionate ethanesulfonate;
31) 2-morpholin-4-yl-ethyl 2-(4-chlorobenzoylamino)-3-(2-oxo-1,2-dihydroquinolin-4-yl)propionate 2-hydroxyethanesulfonate;
32) 2-morpholin-4-yl-ethyl 2-(4-chlorobenzoylamino)-3-(2-oxo-1,2-dihydroquinolin-4-yl)propionate formate;
33) 2-morpholin-4-yl-ethyl 2-(4-chlorobenzoylamino)-3-(2-oxo-1,2-dihydroquinolin-4-yl)propionate galactarate;
34) 2-morpholin-4-yl-ethyl 2-(4-chlorobenzoylamino)-3-(2-oxo-1,2-dihydroquinolin-4-yl)propionate gentisate;
35) 2-morpholin-4-yl-ethyl 2-(4-chlorobenzoylamino)-3-(2-oxo-1,2-dihydroquinolin-4-yl)propionate glutamate;
36) 2-morpholin-4-yl-ethyl 2-(4-chlorobenzoylamino)-3-(2-oxo-1,2-dihydroquinolin-4-yl)propionate glutarate;
37) 2-morpholin-4-yl-ethyl 2-(4-chlorobenzoylamino)-3-(2-oxo-1,2-dihydroquinolin-4-yl)propionate 2-oxoglutarate;
38) 2-morpholin-4-yl-ethyl 2-(4-chlorobenzoylamino)-3-(2-oxo-1,2-dihydroquinolin-4-yl)propionate glycerophosphorate;
39) 2-morpholin-4-yl-ethyl 2-(4-chlorobenzoylamino)-3-(2-oxo-1,2-dihydroquinolin-4-yl)propionate hippurate;
40) 2-morpholin-4-yl-ethyl 2-(4-chlorobenzoylamino)-3-(2-oxo-1,2-dihydroquinolin-4-yl)propionate oleate;
41) 2-morpholin-4-yl-ethyl 2-(4-chlorobenzoylamino)-3-(2-oxo-1,2-dihydroquinolin-4-yl)propionate orotate;
42) 2-morpholin-4-yl-ethyl 2-(4-chlorobenzoylamino)-3-(2-oxo-1,2-dihydroquinolin-4-yl)propionate palmitate;
43) 2-morpholin-4-yl-ethyl 2-(4-chlorobenzoylamino)-3-(2-oxo-1,2-dihydroquinolin-4-yl)propionate pamoate;
44) 2-morpholin-4-yl-ethyl 2-(4-chlorobenzoylamino)-3-(2-oxo-1,2-dihydroquinolin-4-yl)propionate propionate;
45) 2-morpholin-4-yl-ethyl 2-(4-chlorobenzoylamino)-3-(2-oxo-1,2-dihydroquinolin-4-yl)propionate pyroglutamate;
46) 2-morpholin-4-yl-ethyl 2-(4-chlorobenzoylamino)-3-(2-oxo-1,2-dihydroquinolin-4-yl)propionate 4-aminosalicylate;
47) 2-morpholin-4-yl-ethyl 2-(4-chlorobenzoylamino)-3-(2-oxo-1,2-dihydroquinolin-4-yl)propionate sebacate;
48) 2-morpholin-4-yl-ethyl 2-(4-chlorobenzoylamino)-3-(2-oxo-1,2-dihydroquinolin-4-yl)propionate stearate;
49) 2-morpholin-4-yl-ethyl 2-(4-chlorobenzoylamino)-3-(2-oxo-1,2-dihydroquinolin-4-yl)propionate thiocyanate;
50) 2-morpholin-4-yl-ethyl 2-(4-chlorobenzoylamino)-3-(2-oxo-1,2-dihydroquinolin-4-yl)propionate undecylenate;
51) 2-morpholin-4-yl-ethyl 2-(4-chlorobenzoylamino)-3-(2-oxo-1,2-dihydroquinolin-4-yl)propionate hydrobromate;
52) 2-morpholin-4-yl-ethyl 2-(4-chlorobenzoylamino)-3-(2-oxo-1,2-dihydroquinolin-4-yl)propionate isobutyrate;
53) 2-morpholin-4-yl-ethyl 2-(4-chlorobenzoylamino)-3-(2-oxo-1,2-dihydroquinolin-4-yl)propionate laurate;
54) 2-morpholin-4-yl-ethyl 2-(4-chlorobenzoylamino)-3-(2-oxo-1,2-dihydroquinolin-4-yl)propionate DL-mandelate;
55) 2-morpholin-4-yl-ethyl 2-(4-chlorobenzoylamino)-3-(2-oxo-1,2-dihydroquinolin-4-yl)propionate methanesulfonate;
56) 2-morpholin-4-yl-ethyl 2-(4-chlorobenzoylamino)-3-(2-oxo-1,2-dihydroquinolin-4-yl)propionate naphthalene-1,5-disulfonate;
57) 2-morpholin-4-yl-ethyl 2-(4-chlorobenzoylamino)-3-(2-oxo-1,2-dihydroquinolin-4-yl)propionate naphthalene-2-sulfonate;
58) 2-morpholin-4-yl-ethyl 2-(4-chlorobenzoylamino)-3-(2-oxo-1,2-dihydroquinolin-4-yl)propionate 2-naphthoate;
59) 2-morpholin-4-yl-ethyl 2-(4-chlorobenzoylamino)-3-(2-oxo-1,2-dihydroquinolin-4-yl)propionate nicotinate;
60) 2-morpholin-4-yl-ethyl2-(4-chlorobenzoylamino)-3-(2-oxo-1,2-dihydroquinolin-4-yl)propionate nitrate;
61) 2-morpholin-4-yl-ethyl 2-(4-chlorobenzoylamino)-3-(2-oxo-1,2-dihydroquinolin-4-yl)propionate succinate;
62) 2-morpholin-4-yl-ethyl2-(4-chlorobenzoylamino)-3-(2-oxo-1,2-dihydroquinolin-4-yl)propionate sulfonate; and
63) 2-morpholin-4-yl-ethyl 2-(4-chlorobenzoylamino)-3-(2-oxo-1,2-dihydroquinolin-4-yl)propionate L-aspartate.

7. The composition of claim 1, which further contains a carrier, an excipient, a diluent, a filler, an anti-aggregating agent, a lubricant, a wetting agent, a flavoring agent, an emulsifying agent or a preservative.

8. The composition of claim 1, which is formulated in the form of powder, granule, tablet, emulsion, syrup, aerosol, soft or hard gelatin capsule, sterile injectable solution, or sterile powder.

9. The composition of claim 1, which increases tear production and salivary secretion.

10. The composition of claim 1, which decreases mRNA expression levels of immune cell-derived pro-inflammatory cytokines and chemotactic cytokines in lacrimal and salivary gland tissues.

11. The composition of claim 1, which activates survival and growth of cells constituting salivary gland and lacrimal gland tissues.


<110>    SAMJIN PHARM CO LTD

<120>    COMPOSITION FOR PREVENTING OR TREATING SJOGREN'S SYNDROME

<130>    PCT/KR2019/013885

<150>    KR 10-2018-0126875
<151>    2018-10-23

<160>    32

<170>    KoPatentIn 3.0

<210>    1
<211>    20
<212>    DNA
<213>    Artificial Sequence

<220>
<223>    forward primer for IL-2


<400>    1
ttgagtgcca attcgatgat                                                    20


<210>    2
<211>    20
<212>    DNA
<213>    Artificial Sequence

<220>
<223>    reverse primer for IL-2


<400>    2
ttgagggctt gttgagatga                                                    20


<210>    3
<211>    20
<212>    DNA
<213>    Artificial Sequence

<220>
<223>    forward primer for IL-17A


<400>    3
tgtctctgat gctgttgctg                                                    20


<210>    4
<211>    20
<212>    DNA
<213>    Artificial Sequence

<220>
<223>    reverse primer for IL-17A


<400>    4
agtccttggc ctcagtgttt                                                    20

<210>    5
<211>    20
<212>    DNA
<213>    Artificial Sequence

<220>
<223>    forward primer for IFN-g


<400>    5
agctcttcct catggctgtt                                                    20


<210>    6
<211>    20
<212>    DNA
<213>    Artificial Sequence

<220>
<223>    reverse primer for IFN-g


<400>    6
tttgccagtt cctccagata                                                    20


<210>    7
<211>    20
<212>    DNA
<213>    Artificial Sequence

<220>
<223>    forward primer for BAFF


<400>    7
ggtgaccctg ttccgatgta                                                    20


<210>    8
<211>    20
<212>    DNA
<213>    Artificial Sequence

<220>
<223>    reverse primer for BAFF


<400>    8
ctccgttgcg tgaaatctgt                                                    20


<210>    9
<211>    20
<212>    DNA
<213>    Artificial Sequence

<220>
<223>    forward primer for FLT3L

<400>    9
tggcagggtc taagatgcaa                                                    20


<210>    10
<211>    20
<212>    DNA
<213>    Artificial Sequence

<220>
<223>    reverse primer for FLT3L


<400>    10
aggtgggaga tgttggtctg                                                    20


<210>    11
<211>    20
<212>    DNA
<213>    Artificial Sequence

<220>
<223>    forward primer for IL-1


<400>    11
agcttcaaat ctcgcagcag                                                    20


<210>    12
<211>    20
<212>    DNA
<213>    Artificial Sequence

<220>
<223>    reverse primer for IL-1


<400>    12
tctccacagc cacaatgagt                                                    20


<210>    13
<211>    20
<212>    DNA
<213>    Artificial Sequence

<220>
<223>    forward primer for IL-6


<400>    13
gactgatgct ggtgacaacc                                                    20


<210>    14
<211>    20
<212>    DNA
<213>    Artificial Sequence

<220>
<223>    reverse primer for IL-6

<400>    14
agacaggtct gttgggagtg                                                    20


<210>    15
<211>    20
<212>    DNA
<213>    Artificial Sequence

<220>
<223>    forward primer for IL-12A


<400>    15
cctgcactgc tgaagacatc                                                    20


<210>    16
<211>    20
<212>    DNA
<213>    Artificial Sequence

<220>
<223>    reverse primer for IL-12A


<400>    16
ccaggcaact ctcgttcttg                                                    20


<210>    17
<211>    20
<212>    DNA
<213>    Artificial Sequence

<220>
<223>    forward primer for IL-18


<400>    17
gctgccatgt cagaagactc                                                    20


<210>    18
<211>    20
<212>    DNA
<213>    Artificial Sequence

<220>
<223>    reverse primer for IL-18


<400>    18
actgcggttg tacagtgaag                                                    20


<210>    19
<211>    20
<212>    DNA
<213>    Artificial Sequence

<220>
<223>    forward primer for IL-21


<400>    19
agtggcccat aaatcaagcc                                                    20


<210>    20
<211>    19
<212>    DNA
<213>    Artificial Sequence

<220>
<223>    reverse primer for IL-21


<400>    20
aaagctgcat gctcacagt                                                     19


<210>    21
<211>    20
<212>    DNA
<213>    Artificial Sequence

<220>
<223>    forward primer for IL-22


<400>    21
aatcagctca gctcctgtca                                                    20


<210>    22
<211>    20
<212>    DNA
<213>    Artificial Sequence

<220>
<223>    reverse primer for IL-22


<400>    22
tcgccttgat ctctccactc                                                    20


<210>    23
<211>    20
<212>    DNA
<213>    Artificial Sequence

<220>
<223>    forward primer for TNF-alpha


<400>    23
tctcttcaag ggacaaggct                                                    20


<210>    24
<211>    20
<212>    DNA

<213>    Artificial Sequence

<220>
<223>    reverse primer for TNF-alpha


<400>    24
ggcagagagg aggttgactt                                                    20


<210>    25
<211>    20
<212>    DNA
<213>    Artificial Sequence

<220>
<223>    forward primer for IFN-alpha


<400>    25
gctctgtgct ttcctgatgg                                                    20


<210>    26
<211>    20
<212>    DNA
<213>    Artificial Sequence

<220>
<223>    reverse primer for IFN-alpha


<400>    26
gtaccaggag tgtcaaggct                                                    20


<210>    27
<211>    19
<212>    DNA
<213>    Artificial Sequence

<220>
<223>    forward primer for CXCL13


<400>    27
atgaggctca gcacagcaa                                                     19


<210>    28
<211>    20
<212>    DNA
<213>    Artificial Sequence

<220>
<223>    reverse primer for CXCL13


<400>    28
gaataccgtg gcctggagag                                                    20


<210>    29

<211>    20
<212>    DNA
<213>    Artificial Sequence

<220>
<223>    forward primer for CXCR5


<400>    29
ctcaaccgag accttcctgt                                                    20


<210>    30
<211>    20
<212>    DNA
<213>    Artificial Sequence

<220>
<223>    reverse primer for CXCR5


<400>    30
gtgcagagcg atcacagttt                                                    20


<210>    31
<211>    22
<212>    DNA
<213>    Artificial Sequence

<220>
<223>    forward primer for GAPDH


<400>    31
tggagaaacc tgtatgtatg gg                                                 22


<210>    32
<211>    20
<212>    DNA
<213>    Artificial Sequence

<220>
<223>    reverse primer for GAPDH


<400>    32
ggcctgataa gaacaaaccc                                                    20